(19) Europäisches Patentamt
European Patent Office
Office européen des brevets

(11) **EP 2 642 288 A1**

(12) **EUROPEAN PATENT APPLICATION**

(43) Date of publication:
**25.09.2013 Bulletin 2013/39**

(21) Application number: **12160613.1**

(22) Date of filing: **21.03.2012**

(51) Int Cl.:
*G01N 30/96* (2006.01)          *C08B 37/00* (2006.01)
*C12Q 1/56* (2006.01)          *G01N 33/53* (2006.01)
*G01N 33/66* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Istituto di Richerche Chimiche e Biochimiche G. Ronzoni 20133 Milano (IT)**

(72) Inventors:
• **Torri, Giangiacomo**
  **20133 Milano (IT)**
• **Bisio, Antonella**
  **27100 Pavia (IT)**

(74) Representative: **Serravalle, Marco**
  **Serravalle SAS**
  **Via Matteotti, 21/23**
  **26854 Cornegliano Laudense (LO) (IT)**

(54) **Profiling oligosaccharides associated with biological activities of heparin or heparin derivatives**

(57)     The present invention relates to a method for the characterization of heparin or heparin derivatives, comprising isolation and quantification of fractions having different affinities for AT by affinity chromatography on immobilized antithrombin and sub-fractionation of said fractions into size-homogeneous families of oligosaccharides by size-exclusion chromatography (SEC), or into size- and charge- homogeneous families of oligosaccharides by ion pair- reverse phase high performance liquid chromatography (IPRP/HPLC). Fractions without affinity are recovered from the affinity column in isocratic conditions using NaCl 0.05M as eluent, whereas fractions with high affinity are eluted using NaCl ranging from 0.5M to 2.5M. Further physico-chemical validation of heparin and heparin derivatives can comprise homonuclear and heteronuclear correlation spectroscopy. Such method provide analytical profiles relevant to the AT-affinity and the antithrombotic activity of the heparin and LMWH(s).

Fig. 1

EP 2 642 288 A1

Printed by Jouve, 75001 PARIS (FR)

**Description**

BACKGROUND OF THE INVENTION

Heparin, a natural sulfated polysaccharide belonging to the family of glycosaminoglycans (GAG), is widely used in clinics as anticoagulant and, prevalently under the form of low- molecular- weight heparin (LMWH), as antithrombotic drug.[1] Heparin is constituted by linear chains of 1, 4- linked disaccharide repeating units of alternating uronic acids (L-iduronic, IdoA, and D- glucuronic, GlcA) and a hexosamine (D- glucosamine, GlcN) . The prevalent repeating unit in heparins is a trisulfated disaccharide (TSD), where IdoA is O- sulfated at position 2 and GlcN is N- and 6- O sulfated.

. The TSD sequences constitute the so- called regular, "fully sulfated" regions of heparin, which are separated by undersulfated regions bearing a higher content of GlcA with respect to IdoA followed by N- acetyl GlcN (GlcNAc) residues, all with lower content of sulfate groups. Dimeric sequences of different length and sulfation pattern generate chain arrangements responsible for selective protein interactions. The main specific pentasaccharide sequence that binds antithrombin (AT) with high affinity has the following structure: N- acetyl, 6O- sulfated D- glucosamine- D- glucuronic acid- N, 3O, 6O- trisulfated D- glucosamine- 2O- sulfated L- iduronic acid- N, 6O- disulfated D- glucosamine ($A_{NAc,6S}$- G- $A_{NS,3S,6S}$- $I_{2S}$- $A_{NS,6S}$, also indicated as AGA*IA being A* = $A_{NS,3S,6S}$), where $A_{NS/NAc}$ and I residues are in the $\alpha$- configuration and G is in the $\beta$- configuration. It is reported that an IdoA residue is usually present before the AGA*IA sequence in heparin chains.

This sequence is only contained in some (about one third) of the chains constituting the heparins most commonly used in therapy (extracted from pig intestinal mucosa) and even less in the chains of the corresponding LMWHs. It constitutes the antithrombin- binding region (ATBR), which is essential for the inhibition of Factors IIa (thrombin) and Xa leading to the anticoagulant and antithrombotic activity of heparin and LMWHs respectively.[1,2,3]

. Binding of heparin to AT through its ATBR induces a conformational change of the protein that greatly accelerates inhibition of the serine proteases thrombin and Factor Xa and consequently inhibits blood clotting.[1,2,3] Heparin binds also to a number of other proteins[4,5] and modulates fundamental biological functions of its structural analog heparan sulfate (HS), an important constituent of cell surfaces and extracellular matrix.[6,7] For example the prevalent heparin sequence ($I_{2S}$- $A_{NS,6S}$)$_n$, where n $\geq$ 2, preferentially bind to heparin cofactor II and fibroblast growth factor 1, sequence $A_{NS}$- $I_{2S}$- $A_{NS}$- $I_{2S}$ to fibroblast growth factor 2, sequence $I_{2S}$- $A_{NS/NAc}$- G- $A_{NS/NAc}$- $I_{2S}$- $A_{NS/NAc}$ to heparanase and the regular repeating disaccharide units TSD activate platelet factor 4 (PF4) .

Due to size and charge heterogeneity of the natural sources, heparin producers have established their own organ extraction procedures. Similarly, LMWHs manufacturers have optimized processes of depolymerisation to preserve as much as possible the ATBR essential for interaction with AT, as well as a minimal number of adjacent TSD sequences, which play a role in the formation of ternary complex heparin-AT-thrombin.

Intact ATBR is desirable to maximize inhibition of factor Xa and antithrombotic activity of both heparins and LMWHs. However, almost all the (chemical and/or enzymatic) methods of heparin extraction or depolymerisation cleave, at least in part, the pentasaccharidic ATBR sequence. Pentasaccharide sequences AGA*IA surviving the depolymerization process are usually incorporated in LMWH fragments not shorter than hexasaccharide. Octa, deca, and dodecasaccharides containing the AGA*IA at different locations along the oligosaccharide chains were isolated by Sanofi-Aventis from Enoxaparin by preparative ion- exchange chromatography and their conformational and AT- binding properties investigated in collaborative studies with the Ronzoni Institute.[8,9,10,11] Structural variants of AGA*IA (such as N-sulfation instead of N- acetylation of residue A at the non- reducing end of the pentasaccharide sequence, and non- 6-O- sulfation of its residue A*) are known to be compatible with high affinity binding to AT and to the antithrombotic properties.[12] Although not yet disclosed, it is conceivable that oligosaccharide chains of the currently used LMWHs containing fragments of the ATBR, especially the AGA* motif, contribute to different extents to the affinity for AT and associated biological properties of the LMWHs. To be meaningful, structural analysis of LMWHs should accordingly include characterization of oligosaccharides that contain AGA*IA, as well as its variants and fragments.

Most of the current methods for profiling LMWHs disregard the important ATBR-containing components or its variants. For example, while reporting numerous components of the tetra-, hexa- and octasaccharide fractions of Enoxaparin, the Momenta's patent US 7,790,466 B1 does not mention at all those containing the ATBR, therefore confining their analysis to components of LMWHs that are expected to contribute very poorly (if any) to the antithrombotic activity, the major therapeutic indication for this class of drugs.

No analytical method seems to exist for determining contents of intact ATBR characterized by the AGA*IA pentasaccharide.[3] In fact, current methods for determining the compositional characteristics of heparins and LMWHs rely on exhaustive cleavage with heparinases or nitrous acid, which cleave heparin backbones also at the level of the ATBR.[1] Indirectly, the content of ATBR can be derived by determining the content of the 3- O- sulfated glucosamine residue (A*) . This analysis is usually performed by $^{13}$C NMR, chromatographic or capillary electrophoretic (CE) methods, by quantifying (through radioactive detection) A*- containing disaccharides such as $\Delta$UA* or GA*$_{M.ol}$, where $\Delta$U is an unsaturated uronic acid and A*$_{M.ol.}$ is 1, 5- anhydromannitol 3, 6- O- disulfate.[14,15] A* can be determined (by CE) also as part of the tetrasaccharide ΔUAGA* obtained by exhaustive digestion with heparinases.[16,17] Alternatively, the disaccharide GA* can be determined by two- dimensional (2D) NMR directly on heparins and LMWHs.[18]

Although often considered as a marker of ATBR, the presence of A* alone is not sufficient to confer affinity for AT to a heparin sequence. In fact, minor amounts of A* are present also in fractions with no affinity for AT, both in heparins[20] and in LMWHs. [21] Other specific residues within the AGA*IA sequence were found to be essential for high affinity to AT[1-3].

. Although the above parameters contribute to the characterization of heparin and heparin derivatives, it must be emphasized that they do not differentiate among ATBR sequences present in chains of different length nor their definite position in each chain. On consideration of the significant impact of the length of chains and of the location of ATBR along each chain on the affinity for AT and antithrombotic activity,[8] this is an important limitation in the characterization of biologically-relevant sequences of heparins and LMWHs. Especially for bioanalytical profiles of LMWHs, it is now clear that determination of the actual structure and relative content of their major constituting oligosaccharides as described hereafter is essential.

## BRIEF DESCRIPTION OF FIGURES

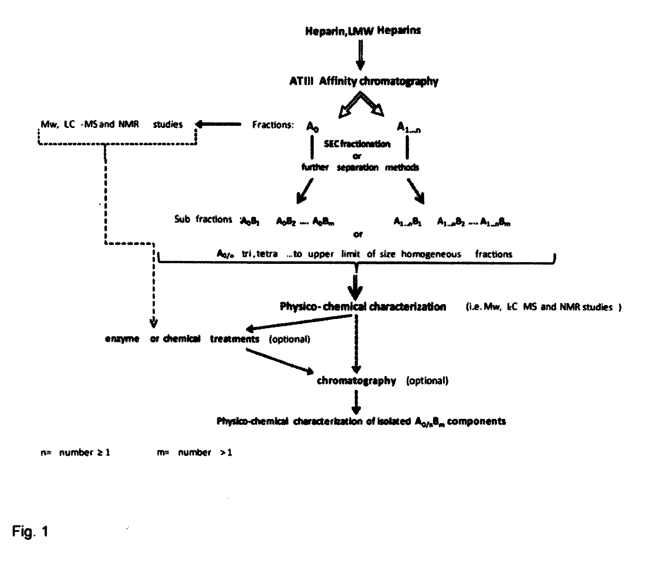

Figure 1: representative scheme of the method applied for the characterization of heparin and heparin derivatives.

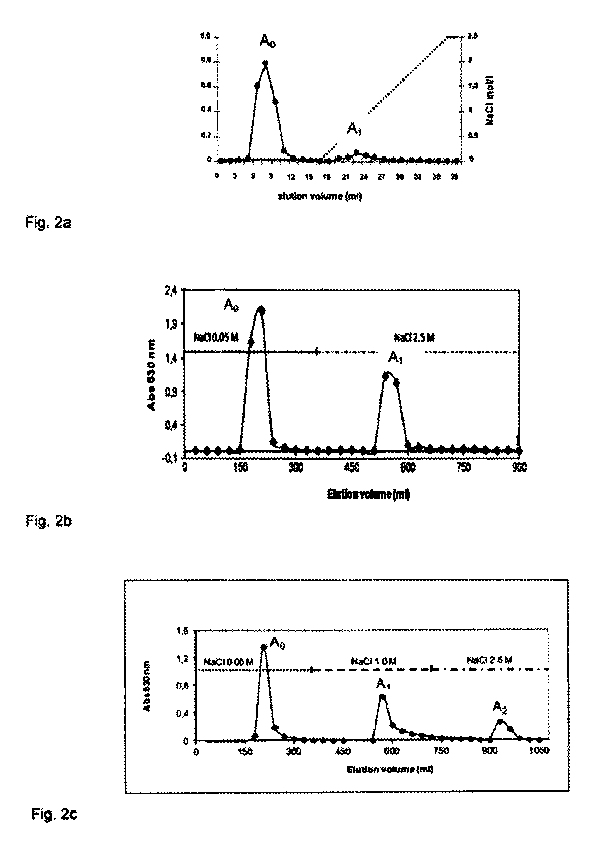

. Figures 2a, 2b and 2c: Affinity chromatography on AT-Sepharose of Lovenox in different experimental conditions: a) delayed continuous salt gradient, b) two-step salt gradient, c) three-step salt gradient. The salt concentration used for elution is indicated by the broken line. In all cases $A_0$ fraction is eluted in isocratic condition at NaCl 0.05 M.

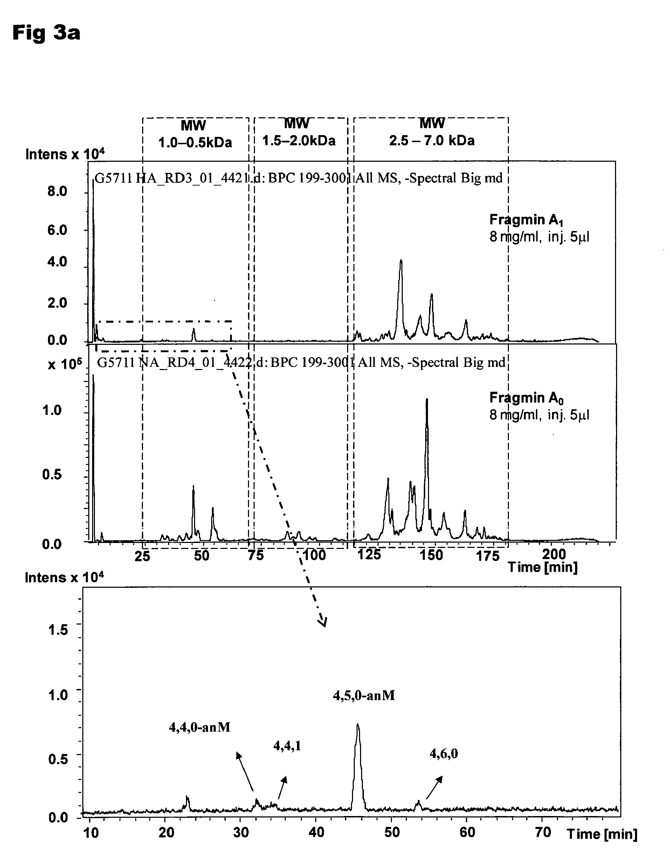

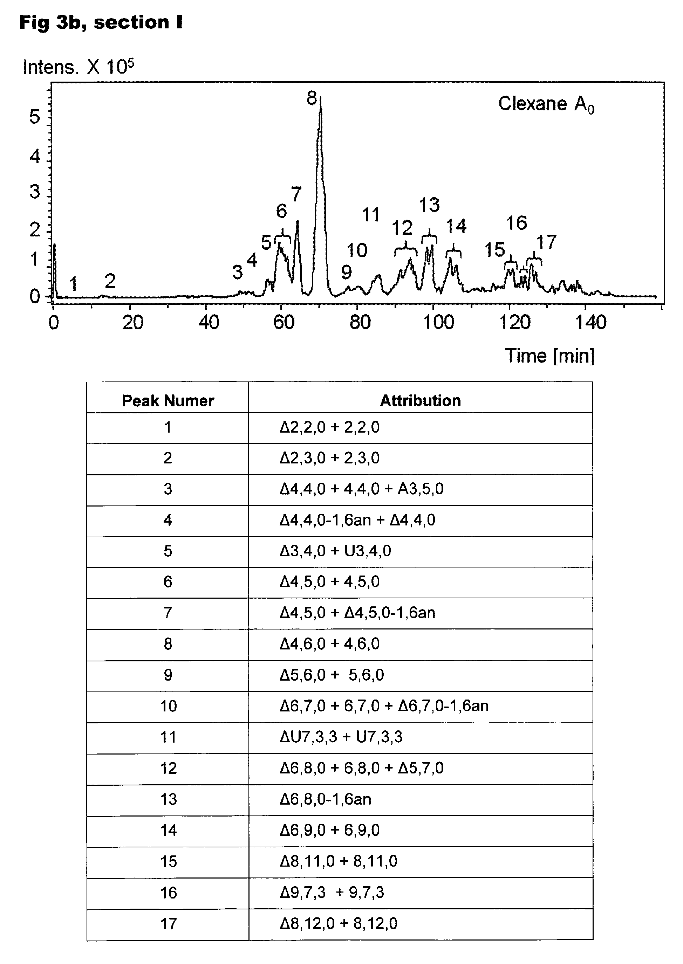

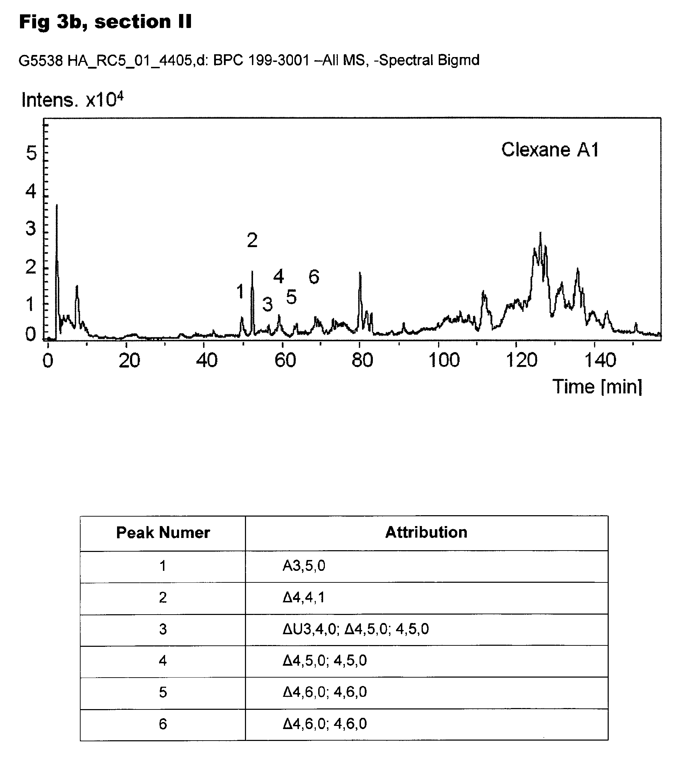

Figures 3a and 3b: TIC RP-IP-HPLC/MS profiles of fractions $A_0$ and $A_1$, obtained by affinity fractionation according to the two-step salt gradient elution method, of (a) Dalteparin, with vertical expansion of minor tetrameric components of $A_1$ and (b) Clexane. The MS spectrum of each single peak allows the determination of the mass of the components. Δ and the following three numbers indicate the 4,5 double bond, the number of monomers, the number of sulfate groups and the number of acetyl groups respectively, anM indicates anhydromannitol residue. Quantitative evaluations can be performed using different appropriate calibration standards. All the identified tetramers of both $A_1$ fractions are compatible with sequences containing the ATBR fragments.

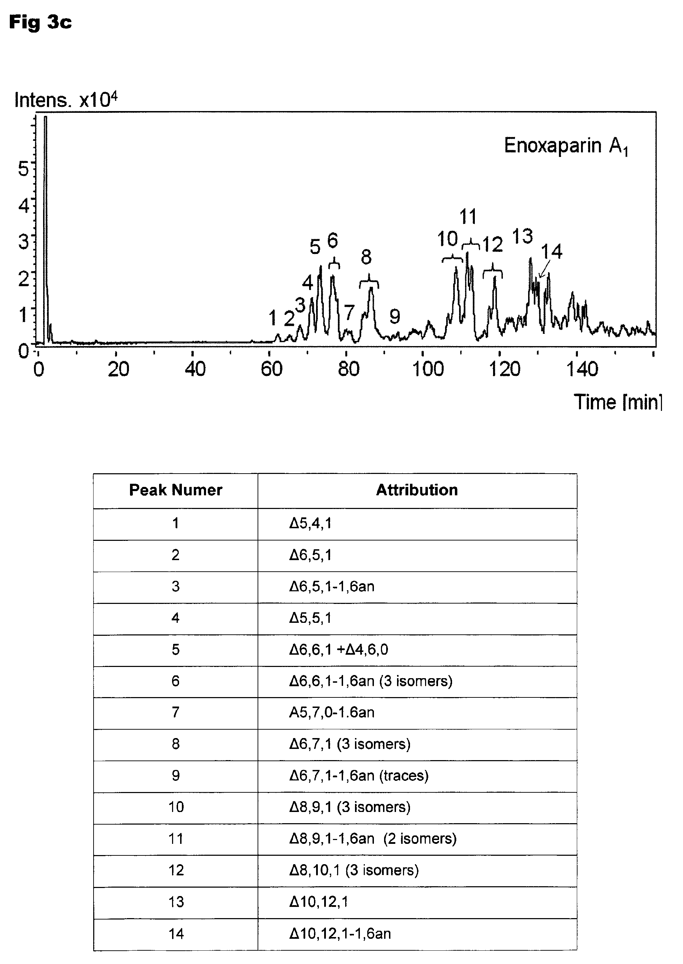

Figure 3c: Chromatographic profiles of $A_1$ fractions of Enoxaparin obtained by affinity fractionation according to the three-step salt gradient elution method.

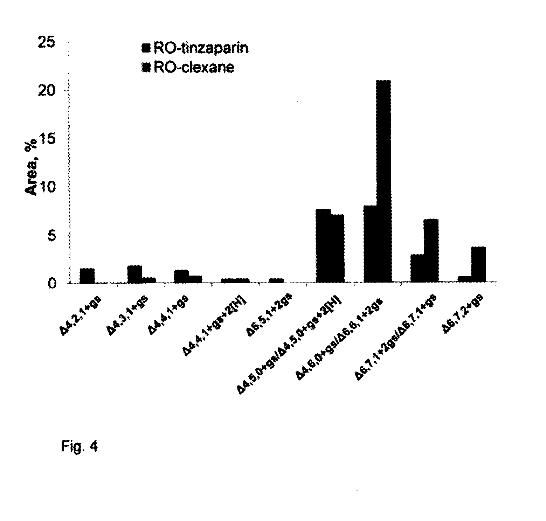

. Figure 4: RP- IP- HPLC/MS analysis of An fractions exhaustively digested with heparinases after glycolsplitting reaction. The different relative proportions of families of glycol- split (gs) tetra- and penta- sulfated tetrasaccharides, and hexa- and hepta- sulfated hexasaccharides depend on the type of LMWH. Some of the structures (i.e.: Δ4, 6, 0+gs, Δ6, 7, 1+2gs) are components derived from the ATBR sequence or its fragments and can be considered as markers of it.

Figure 5: Size exclusion chromatography (SEC) elution profile on Biogel P6 of Lovenox.

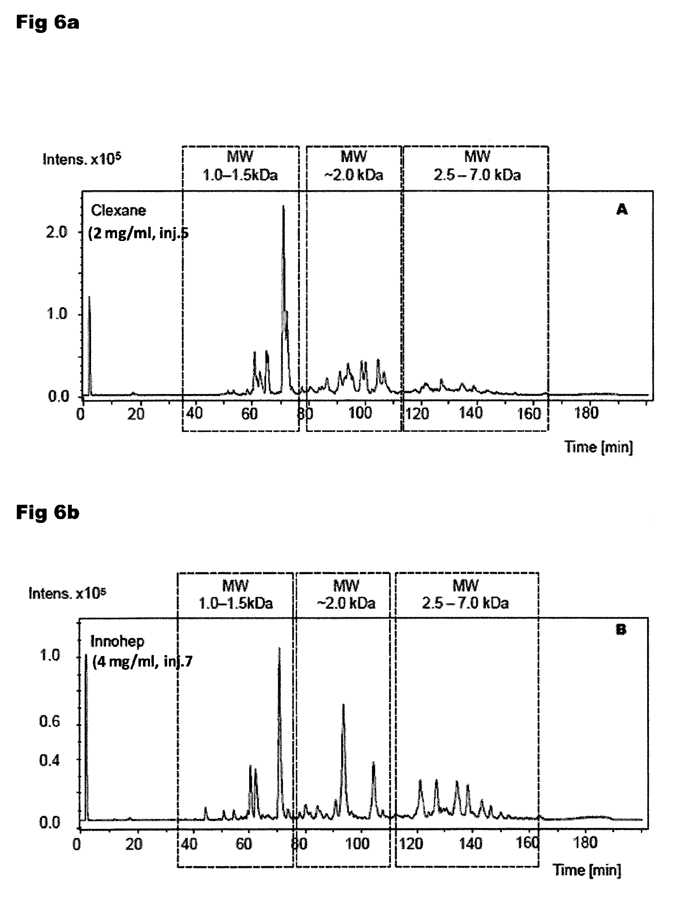

Figure 6a and 6b: RP-IP HPLC/MS chromatographic profiles of Clexane (a) and Innohep (b).

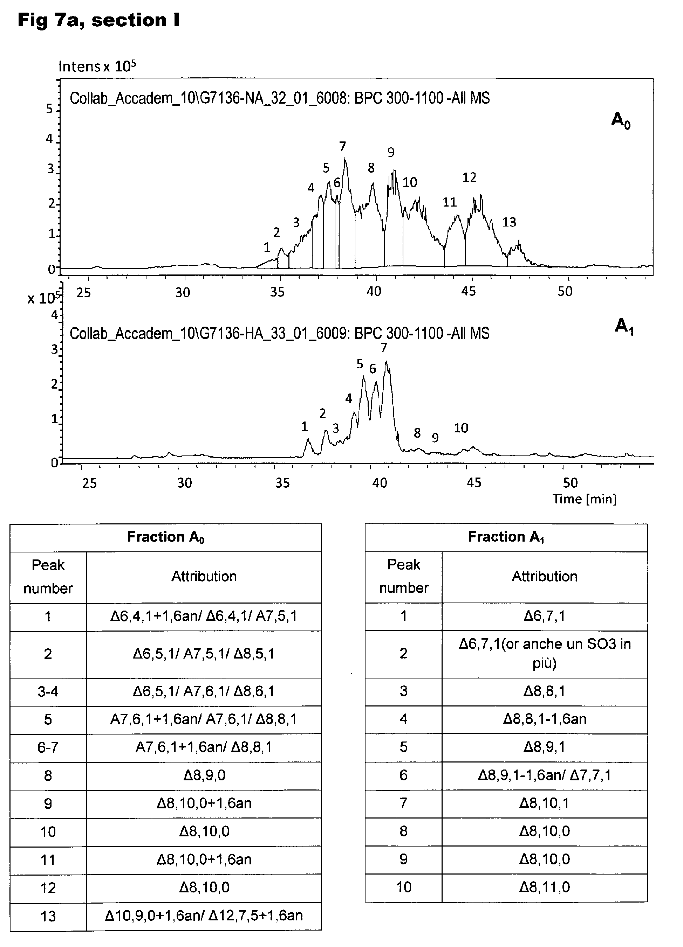

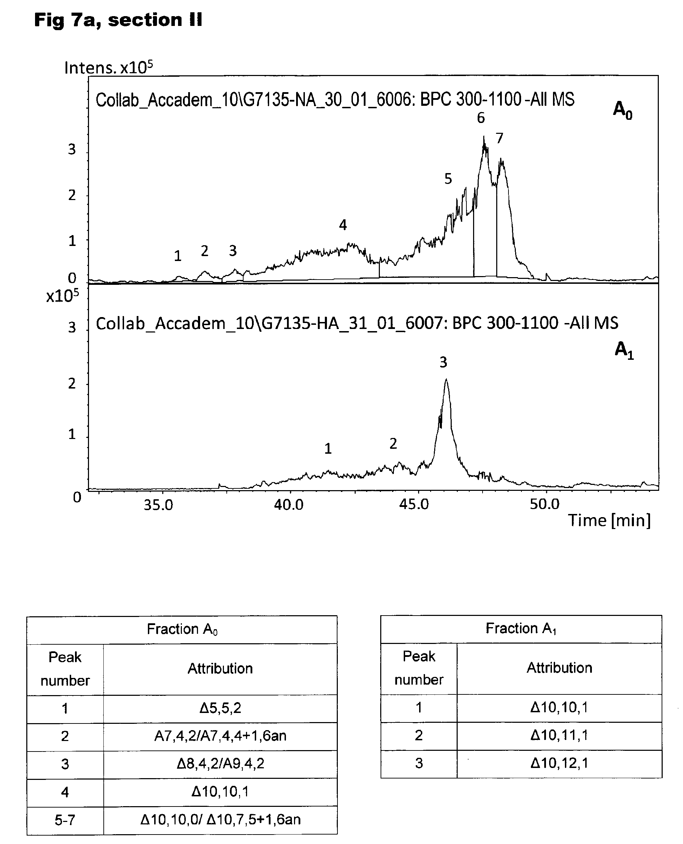

Figure 7a: RP- IP- HPLC/MS profiles of $A_0$ and $A_1$ fractions of Clexane octa- (upper panel) and deca- saccharide (lower panel) . The mass values of major peaks correspond to oligosaccharides containing the intact I- AGA*IA sequence. In particular, the major peak of the $A_1$ octasaccharide sub- fraction (Δ8, 10, 1) is compatible with the structure $\Delta U_{2S}$-$A_{NS,6S}$- I- $A_{NAc,6S}$- G- $A_{NS,3,6S}$- $I_{2S}$- $A_{NS,6S}$ (OCTA- 3 of *ref.* 8), and the major peak of the $A_1$ decasaccharide sub- fraction (Δ10, 12, 1) is compatible with the structure of OCTA- 3 prolonged by a disulfated disaccharide unit. The present assignments are also supported by the finding of Mourier and Viskov who established the above structure for the prominent AGA*IA- containing octa- and decasaccharide component of heparin.[19] The figures also effectively illustrate the unique effectiveness of RP- HPLC/MS profiles in distinguishing $A_0$ from $A_1$ oligosaccharides and show for the first time that oligosaccharides containing the "signature groups" 1, 6- anhydro are almost exclusively contained in the subfraction with no affinity for AT.

Figure 7b: RP- IP- HPLC/MS profiles of $A_0$ and $A_1$ fractions of Innohep octa- (upper panel) and deca- saccharide (lower panel) . The mass values of major peaks correspond to oligosaccharides containing the intact hexasaccharide I- AGA*IA sequence, i.e., the active pentasaccharide AGA*IA preceded by an IdoA residue as usual in the ATBR of heparins. In particular, the major peak of the $A_1$ octasaccharide sub- fraction (Δ8, 9, 1) is compatible with the structure $\Delta U_{2S}$- $A_{NS,6S}$- I- $A_{NAc,6S}$- G- $A_{NS,3,6S}$- $I_{2S}$- $A_{NS,6S}$.

Figure 7c: RP-IP-HPLC/MS profiles of $A_0$ and $A_1$ fractions of Fragmin octasaccharide.

Figure 8: HSQC spectra anomeric region of Fragmin $A_1$ and $A_0$ decasaccharides. In the latter component, the presence inside the chain of a ring contracted unit derived from an hexosamine residue is highlighted ($A_x$).

Figure 9: Anomeric region of $^1$H- NMR spectra of $A_0$ tetrasaccharides isolated from Lovenox. (A) single hexasulfated species; (B) two pentasulfated $A_0$ tetrasaccharide sequences, differing for the epimerization of the reducing terminal hexosamine, i.e. N- sulfate 6- O- sulfate glucosamine and N- sulfate 6- O- sulfate mannosamine; (C) two

pentasulfated $A_0$ tetrasaccharide sequences, differing for the epimerization of the reducing terminal hexosamine, i.e. 1, 6- anhydro- N- sulfate glucosamine and 1, 6- anhydro- N- sulfate mannosamine.

## DESCRIPTION OF THE INVENTION

The present invention relates to a method for the characterization of heparin or heparin derivatives, such as LMWHs, preferably through the identification and quantification of the GA*, regarded as the minimal structural requirement (minimal AT binding requirement, mATbr) of ATBR or its variants for binding AT, in size homogeneous or in charge and size homogeneous oligosaccharides, or in single oligosaccharide component. Examples of said heparin derivatives are Lovenox, Clexane, Fragmin, Innohep, Nadroparin, Parnaparin, and the non-proprietary versions based on their active ingredient Enoxaparin, Dalteparin, Tinzaparin, Fenton-type heparin derivatives[22] and their oligosaccharidic components. Such method provides analytical profiles relevant to the AT-affinity and the antithrombotic activity of the heparin and LMWH(s). We discovered that oligosaccharides containing not yet described variants of the pentasaccharide AGA*IA bind AT in the AT-affinity column. The method disclosed in the present invention is more suitable than the currently accepted methods for establishing the sameness of generic LMWHs with respect to their originator compounds.

The "active" anticoagulant/antithrombotic chains, i.e., those containing the ATBR as AGA*IA and its AT-binding variants, fragments or extended AGA*IA, can be separated from the bulk of chains constituting heparins and LMWHs by affinity chromatography on immobilized AT[13]. The fraction without affinity is recovered in isocratic condition using NaCl 0.05M as eluent, whereas fraction(s) with high affinity is/are eluted using NaCl ranging from 0.5M to 2.5M. The relative content of fractions with high affinity and of those with no affinity is a useful parameter to characterize heparins and LMWHs.

Size homogeneous oligosaccharides not containing the mATbr sequence are characterized as well in order to identify other sequences relevant for biological activities or for side effects in heparin- based treatments.

Although often considered as a marker of ATBR, the presence of A* alone is not sufficient to confer affinity for AT to a heparin sequence. Minor amounts of A* are present also in fractions with no affinity for AT, both in heparins[20] and in LMWHs.[21] In fact, other specific residues within the AGA*IA sequence were found to be essential for high affinity to AT[1-3]. We discovered that oligosaccharides containing not yet described variants of the pentasaccharide AGA*IA bind AT in the AT-affinity column.

We have shown that the disaccharide GA*, can be present in different sequences according to the following general formula 1:

$$[X]_a - [Y]_b - G - A^* - [Z]_c - [W]_d$$

where:

X is an uronic acid including the 4, 5 unsaturated residues; Y is a glucosamine; Z is an uronic acid; W is a NS glucosamine. X and Z , as well as Y and W, can present the sulfation patterns of uronic acids and glucosamines typical of glycosaminoglycans family.

a, c, b and d are numbers with the following limitations:

c, b, are comprised from 0 to 30;

a = b or = b-1; d = c or = c-1

and

when b = 0 and a = 0, c≥1 and d is comprised from c and c-1

when c = 0 and d = 0, b≥1 and a is comprised from b and b-1

The novel analytical approach relies on a step of separation preliminary to further fractionations of heparin and heparin derivatives. The preliminary separation step of heparin or LMWHs or their fractions is performed by affinity chromatography on immobilized AT to separate and isolate components having no affinity and significant affinity for AT. Such components are collected at least in two ($A_0$, $A_1$) or more fractions ($A_0$, $A_1$, $A_2$, ...An) according to different affinity strengths for AT (see scheme in fig. 1), wherein the fraction without affinity ($A_0$) is recovered in isocratic condition using NaCl 0.05M as eluent, whereas fraction (s) with high affinity is/are eluted using a continuous gradient ($A_1$), or two or more step gradient ($A_1$, $A_2$, ...$A_n$) using NaCl ranging from 0.05M to 2.5M (see fig. 2a, b, c) .

The quantitative distribution of fractionated components depends on the column size, on the volume of eluted fractions, and on the adopted methods (i.e. continuous gradient vs step gradient (s) ) . Table 1a reports the content of high affinity fraction ($A_1$) of different LMWHs (commercial and pilot scale preparations) obtained by two different methods on an analytical column. The percentage of fraction $A_1$ was determined according to continuous salt gradient elution method or according to two- step salt gradient elution method. The reproducibility of both methods was proved by running 6 or 5 times the same sample. Table 1b reports the content of high affinity fractions ($A_1$ and $A_2$) obtained with three- step salt gradient elution method on a preparative column.

Table 1a: Relative content of the $A_1$ fraction, evaluated as weight %, in different LMWH preparations isolated by affinity chromatography on analytical column with continuous and two step salt gradient elution.

| Sample | N° of experiments | Continuous gradient % $A_1$ | Two step gradient % $A_1$ |
|---|---|---|---|
| | | | |
| Lovenox *a* | 3 | 12 7 $\pm$ 0 75 | |
| Lovenox *b* | 3 | 12 8 $\pm$ 1 97 | |
| Lovenox *c* | 3 | 13 4 $\pm$ 0 87 | |
| Lovenox *d* | 3 | | 14 9 $\pm$ 0 57 |
| Lovenox *e* | 3 | | 10 6 $\pm$ 0 93 |
| | | *Range· 9 6 - 15 4%* | |
| Clexane *a* | 3 | | 13 2 $\pm$ 0 72 |
| Clexane *b* | 3 | | 12 9 $\pm$ 0 35 |
| | | *Range· 12 4 - 13 6%* | |
| Enoxaparin *a* | 6 | 10 4 $\pm$ 0 61 | |
| Enoxaparin *b* | 3 | 9 4 $\pm$ 0 30 | |
| Enoxaparin *c* | 3 | 9 6 $\pm$ 0 68 | |
| Enoxaparin *d* | 4 | | 10 0 $\pm$ 0 44 |
| Innohep | 5 | | 22 5 $\pm$ 2 06 |
| Fragmin | 3 | | 15 1 $\pm$ 0 43 |
| Nadroparin | 3 | | 13 9 $\pm$ 2 20 |
| Fraxiparin | 3 | | 18 2 $\pm$ 0 98 |

Table 1b: Relative content of the affinity fractions, $A_1$ and $A_2$, evaluated as weight %, isolated from three batches of Lovenox by affinity chromatography on preparative column with three step salt gradient elution.

| | Three step gradient on preparative column | | |
|---|---|---|---|
| | N°of experiments | % $A_1$ | % $A_2$ |
| | | | |
| Lovenox *f* | 6 | 21 5 $\pm$ 2 19 | 4 4 $\pm$ 1 04 |
| Lovenox *g* | 4 | 19 8 $\pm$ 3 58 | 4 5 $\pm$ 1 51 |
| Lovenox *h* | 3 | 21 7 $\pm$ 0 30 | 5 3 $\pm$ 2 38 |

In this approach oligosaccharides containing intact ATBR fragments with AGA*IA or G-A* sequence can be collected in high affinity fractions ($A_1$ An). Such a fractionation permits i) to increase the number of analytical parameters useful for characterizing and comparing different components of heparin and LMWH preparations; ii) to reduce the complexity of $A_0$ fraction components with respect to the original not fractionated LMWH, iii) to reduce the complexity of $A_1$ fraction components, when n is >1 and to facilitate the analysis and the consequent characterization and quantification of oligosaccharidic components.

. In one embodiment the obtained fractions are profiled as such and optionally after sodium metaperiodate oxidation followed by a treatment with a reduction agent to generate glycol- split non sulfated uronic acid residues.[23] The profile is obtained using an optimized high resolution LC- MS/UV technique, such as reverse phase- ion pair high performance liquid chromatography on line with mass spectrometry (RP- IP- HPLC/MS), as in fig. 3, or strong anion exchange HPLC- MS (SAX- HPLC off line with MS), and by quantitative mono and bi dimensional homo and hetero NMR spectroscopy[24,25,26,27,28,28,29] to obtain characteristic distributions of oligosaccharide chains and the quantification of all detectable mono- disaccharide residues, respectively (currently identified signals are reported in table 4) . Such detailed characterization, using different physico- chemical methods, of components of heparins and LMWH (s) (including those contained in fractions with no affinity for antithrombin oligosaccharides with odd numbers of residues, modified residues such as 2, 6 anhydro hexosamines, uronic acid epoxides, galacturonic acid residues and ring contracted unit derived from hexosamine residues) permits correlations also with biological activities elicited by proteins other than AT. Furthermore, the SAX or RP- IP- HPLC analysis of An fractions exhaustively digested with heparinases after the glycol splitting reaction permits the identification of the glycol- split residues corresponding to nonsulfated uronic acid residues along the original heparin (LMWH) chains, and an useful profiling of resulting oligosaccharides together with the identification of the components derived from the ATBR sequence that can be considered as marker of it, e.g. $\Delta$4, 6, 0+gs, $\Delta$6, 7, 1+2gs (fig. 4) .

In another embodiment further physico-chemical characterizations comprise absolute molecular weight ($M_W$, Mn, $M_{iv}$ and polydispersity) determinations. The approach is based on a refined SEC chromatography methodology coupled with light scattering and/or viscometer/refractive index detectors and adequate software for the evaluations of data.[30] This procedure does not need any heparin/LMWH Mw calibrators.

The oligosaccharides of $A_{0/n}$ fractions obtained by the preliminary separation step/s are further separated on the bases of molecular size, this step comprising size exclusion chromatography (SEC), RP-IP chromatography and obtaining $A_0$-oligo and $A_n$-oligo ($A_{0/n}B_m$) fractions (as schematized in figure 1). In a preferred embodiment, but not excluding the use of the other conventional automatic approaches, the fractionation is performed by SEC on a column of at least 1.6 m length and 2 to 5 cm of diameter, using low pressure conditions on commercial resins such as Biogel P6 and P10, depending on the need.

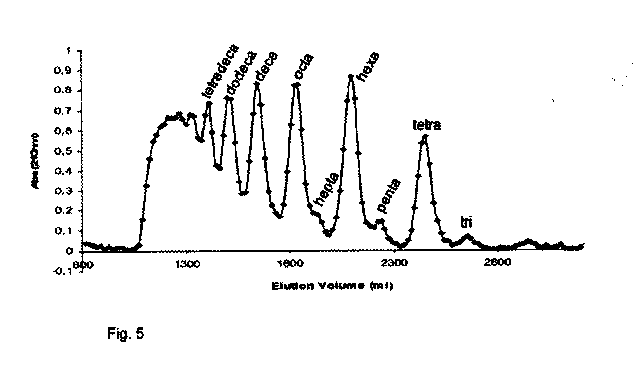

Such SEC method aims at a very detailed compositional analysis of heparin and LMWH(s) and is based on highly resolving chromatographic approaches. SEC was indeed optimized to achieve an improved separation of oligosaccharidic components which permits to isolate, as in the case of Clexane, Lovenox and enoxaparin, also odd oligomers such as tri and pentamers (fig 5). Such chromatographic efficiency is guaranteed by a number of theoretical plates not lower than 6000 for the tetrasaccharidic components. Accordingly, the quantification of separated components is more precise with respect to methods already reported (tables 2a and 2b). In another embodiment the RP-IP chromatography is used to separate oligosaccharidic components.

Table 2a: Relative abundance of the oligosaccharidic components of Lovenox, Innohep and Fragmin determined by size exclusion chromatography on Biogel P6. The amounts are determined by as area under the curve, evaluated at 210 nm. The number of experiments (n) per each LMWH is indicated.

| oligosaccharide | Lovenox n=7 | | Innohep n=5 | | Fragmin n=6 | |
|---|---|---|---|---|---|---|
| | area % | | area % | | area % | |
| dp2-dp3 | 2.0 | ± 1.01 | 0.6 | ± 0.59 | nd | nd |
| dp4 | 8.9 | ± 2.09 | 3.9 | ± 1.11 | nd | nd |
| dp5 | 2.6 | ± 1.42 | nd | nd | nd | nd |
| dp6 | 12.4 | ± 0.76 | 8.4 | ± 2.46 | nd | nd |
| dp8 | 14.1 | ± 0.96 | 8.2 | ± 0.36 | 3.7 | ± 0.93 |
| dp10 | 12.5 | ± 1.36 | 9.1 | ± 0.6 | 6.2 | ± 0.50 |
| dp12 | 10.6 | ± 0.78 | 7.7 | ± 2.11 | 8.7 | ± 0.73 |
| dp14 | 9.5 | ± 1.28 | 7.1 | ± 1.29 | 9.7 | ± 0.66 |
| dp>14 | 27.4 | ± 2.43 | 55.0 | ± 2.20 | 71.6 | ± 1.55 |
| nd: not detectable | | | | | | |

| Lovenox | | | From Patent US 7,790,466 B1 | | |
|---|---|---|---|---|---|
| | | | Area % | | |
| oligosaccharide | area % | Range | Range A | Range B | Range C |
| dp2-dp3 | 1.2 ± 0.91 | 0.2 - 2.6 | nr | nr | nr |
| dp4 | 8.6 ± 1.89 | 6.5 -11.6 | 9.8 - 12.3 | 9.1 - 12.5 | 8.0 - 13.7 |
| dp5 | 2.2 ± 1.44 | 0.8 - 4.7 | nr | nr | nr |
| dp6 | 12.9 ± 0.80 | 11.6 - 13.7 | 18.8 - 20.4 | 18.4 - 20.6 | 17.6 - 21.3 |
| dp8 | 14.4 ± 0.67 | 13.5 - 15.0 | 16.8 - 17.4 | 16.6 - 17.5 | 16.3 - 17.9 |
| dp10 | 12.7 ± 0.93 | 11.0 - 13.7 | 13.0 - 14.1 | 12.9 - 14.4 | 12.4 - 14.9 |
| dp12 | 11.2 ± 0.71 | 10.2 - 12.1 | | | |
| dp14 | 9.2 ± 0.98 | 7.5 - 10.2 | | 33.4 - 39.9 | 31.3 - 42.0 |
| dp>14 | 27.8 ± 2.34 | 25.1 - 30.4 | 34.0 - 38.8 | | |

nr: not reported

Table 2b: Relative abundance of the oligosaccharidic components of six different batches of Lovenox determined by size exclusion chromatography on Biogel P6. The amounts are expressed as area under the curve, evaluated at 210 nm. For each oligosaccharide, together with area % and the relative standard deviation, the range in area % is also reported. Data from Patent US,7,790,466 B1 are reported for comparison.

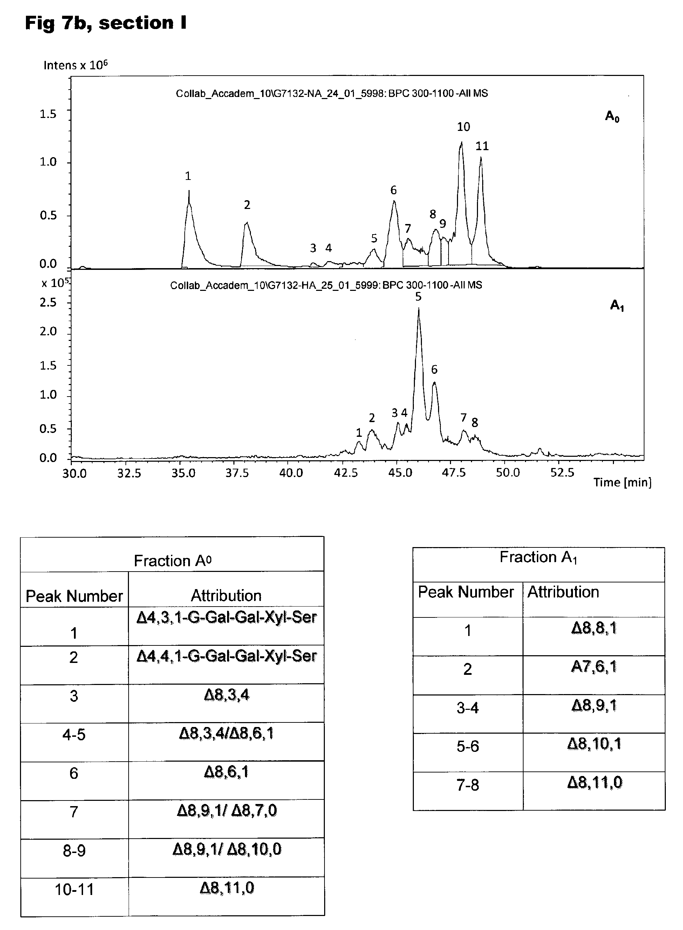

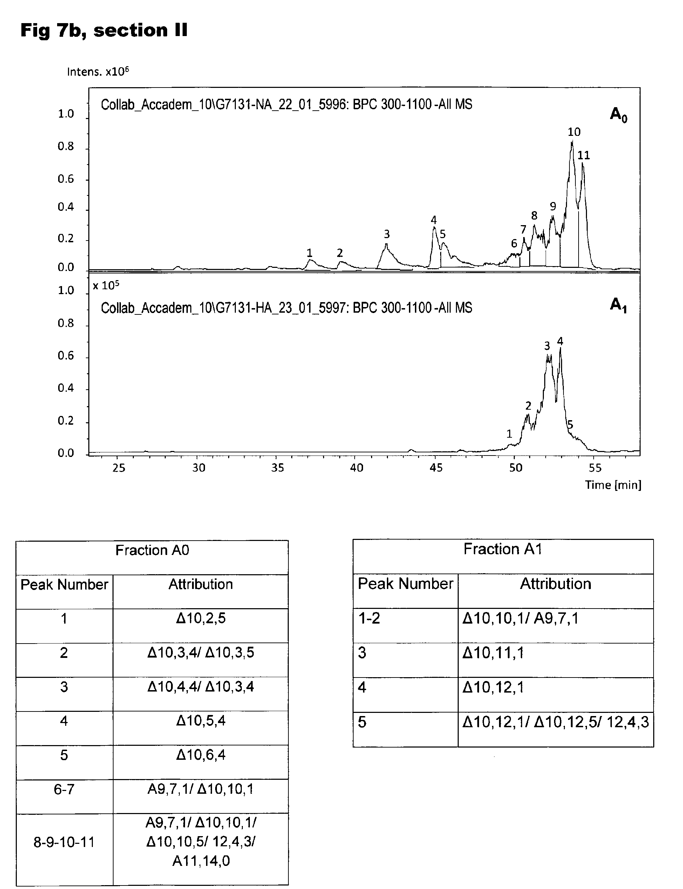

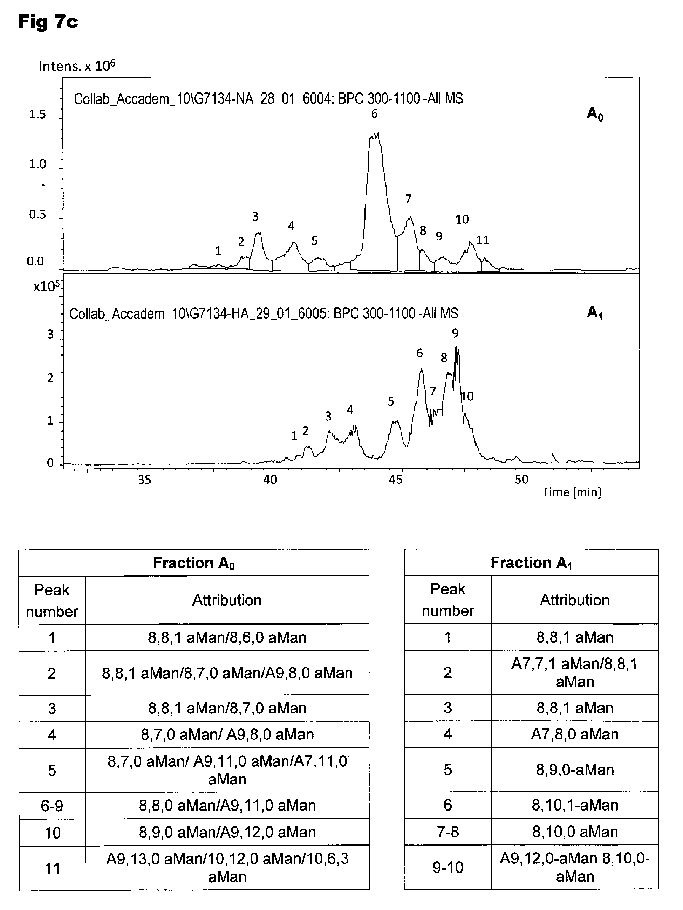

In a preferred embodiment, the $A_{0/n}B_m$ fractions are further separated using RP-IP-HPLC apparatus on- or off-line with ESI-IT-MS/UV spectrometers and/or with NMR and/or other high accuracy MS and/or MS/MS spectrometers. Such a combination of techniques allows to achieve good resolution and detection sensitivity of oligosaccharide constituents, including the odd and even ones, from tri/tetra- to deca- saccharides or longer oligosaccharide chains (fig.7).

In another preferred embodiment of the invention, in between the affinity chromatography and SEC or RPIP-HPLC steps, a further separation by ion exchange chromatography is performed. Other but not exclusive fractionation procedures for this further step are based on fractionated precipitation, electrophoresis, affinity for heparin binding proteins different from AT.

Size- homogeneous subfractions of LMWHs with high affinity ($A_nB_m$) and no affinity ($A_0B_m$) for antithrombin are profiled or fractionated, in a preparative column, by RPIP LC/MS. The number of chromatographic peaks of the $A_1B_m$ fractions are consistently lower than for the $A_0B_m$ fractions, being their mass values different from those of the corresponding $A_0B_m$ fractions. In particular, the relative intensity of major peaks of the $A_nB_m$ fractions assigned to oligosaccharides, which likely contain the essential part of the ATBR (i.e., at least the sequence G- A*) is compared with those of other peaks of the $A_nB_m$ and $A_0B_m$ fractions (tables 3 a and b) . The intensity peak ratios for components of size-homogeneous $A_{1,octa/deca}$ and $A_{0,octa/deca}$ sub- fractions are highly characteristic of individual LMWHs. These parameters are also in line with the contents of the marker disaccharide G- A* as determined by NMR spectroscopy (tables 4 a and b) . This technique, applied to the isolated components, obtained by preparative procedure, allows to identify the structural variant of the ATBR sequence.

Table 3a: Composition and area % recovery (TIC) of components of Innohep high affinity (A$_1$) octasaccharidic fractions obtained by RP-IP-HPLC-/MS analysis. The structures are indicated as number of residues, numbers of sulphate groups, numbers of acetyl groups; the symbol $\Delta$ indicates an unsaturation on uronic acid at non-reducing end. N indicates the peak number.

| Innohep octasaccharides, A$_1$ fraction | | | |
|---|---|---|---|
| N | Compound | Area, S.10$^{-6}$ | Area, % |
| 1 | $\Delta$8,8,1 | 0.5 | 3.0 |
| 2 | A7,6,1 | 1.7 | 10.4 |
| 3 | $\Delta$8,9,1 | 1.0 | 6.1 |
| 4 | $\Delta$8,9,1 | 1.0 | 6.4 |
| 5 | $\Delta$8,10,1 | 6.2 | 38.0 |
| 6 | $\Delta$8,10,1 | 3.5 | 21.3 |
| 7 | $\Delta$8,11,0 | 1.1 | 6.9 |
| 8 | $\Delta$8,11,0 | 1.2 | 7.8 |

Table 3b: Composition and area % recovery (TIC) of components of Clexane high affinity (A$_1$) octasaccharidic fractions obtained by RP-IP-HPLC-/MS analysis. The structures are indicated as

| Clexane octasaccharides, A$_1$ fraction | | | |
|---|---|---|---|
| N | Compound | Area, S.10$^{-6}$ | Area, % |
| 1 | $\Delta$6,7,1 | 1.4 | 3.4 |
| 2 | $\Delta$6,7,1 or $\Delta$6,8,1 | 2.4 | 6.2 |
| 3 | $\Delta$8,8,1 | 2.3 | 6.2 |
| 4 | $\Delta$8, 8, 1- 1, 6an | 2.9 | 7.3 |
| 5 | $\Delta$8,9,1 | 6.6 | 17.3 |
| 6 | $\Delta$8, 9, 1- 1, 6an/ $\Delta$7,7,1 | 6.0 | 15.4 |
| 7 | $\Delta$8,10,1 | 10.7 | 28.1 |
| 8 | $\Delta$8,10,0 | 1.3 | 4.8 |
| 9 | $\Delta$8,10,0 | 1.0 | 4.4 |
| 10 | $\Delta$8,11,0 | 1.8 | 6.8 |

number of residues, numbers of sulphate groups, numbers of acetyl groups; the symbol $\Delta$ indicates an unsaturation on uronic acid at non-reducing end. N indicates the peak number.

| | $A_{NS}-I_{2S}$ | $A_{NS}-I$ | $A_{NS}-G$ | $A_{NS,3S,6S}(A^*)$ | $A_{NAc}-G$ | $A_{NAc}-I$ | $A-GalA$ | $A_{NS}red\alpha + A^*red$ | $A_{NS}red\beta$ | $A_{NAc}red\alpha$ | $A_{NH2}$ | $A_{6S}$ | 1,6 an A | 1,6 an M | $M_{NS\,\alpha-red}$ | An.M.ol | Ax |
|---|---|---|---|---|---|---|---|---|---|---|---|---|---|---|---|---|---|
| **Clexane** | | | | | | | | | | | | | | | | | |
| deca | 45.9 | 8.4 | 12.6 | **3.6** | 9.1 | 0.4 | 3.1 | 8.6 | 0.7 | 0.6 | 0.0 | 86.0 | 2.3 | 2.6 | 2.2 | 0 | 0 |
| deca $A_1$ | 21.9 | 13.6 | 11.8 | **18.9** | 15.4 | 0.7 | 1.3 | 7.9 | 1.1 | 0.7 | 0.7 | 95.0 | 2.1 | 1.9 | 2.1 | 0 | 0 |
| deca $A_0$ | 47.9 | 7.5 | 13.7 | **1.6** | 8.6 | 0.3 | 4.1 | 7.1 | 0.9 | 0.5 | 1.5 | 83.5 | 2.5 | 2.5 | 1.6 | 0 | 0 |
| octa | 49.1 | 4.4 | 13.9 | **3.4** | 3.6 | 0 | 4.8 | 9.5 | 0.9 | 0 | 0 | 88.6 | 3.3 | 3.6 | 3.6 | 0 | 0 |
| octa $A_1$ | 16.1 | 11.2 | 0 | **25.3** | 21.7 | 0 | 0 | 17.9 | 0 | 0 | 0 | 94.6 | 0 | <1 | 7.7 | 0 | 0 |
| octa $A_0$ | 50.5 | 3.8 | 11.0 | **1.6** | 5.1 | 0 | 2.7 | 9.2 | 0.7 | 0 | 0 | 88.6 | 9.2 | 3.1 | 3.1 | 0 | 0 |
| **Innohep** | | | | | | | | | | | | | | | | | |
| deca | 56.0 | 7.0 | 8.8 | **2.7** | 7.1 | 0.4 | 0 | 14.5 | 2.0 | 0.6 | 1.0 | 87.2 | 0 | 0 | 0 | 0 | 0 |
| deca $A_1$ | 24.3 | 20 | 5.6 | **17.2** | 15.4 | 0 | 0 | 17.4 | 0 | 0 | 0 | 87.1 | 0 | 0 | 0 | 0 | 0 |
| deca $A_0$ | 55.5 | 6.6 | 9.3 | **1.2** | 8.4 | 0.5 | 0 | 12.8 | 2.9 | 0.6 | 2.2 | 87.1 | 0 | 0 | 0 | 0 | 0 |
| octa | 53.5 | 6.3 | 8.5 | **1.9** | 5.9 | 0.5 | 0 | 18.7 | 2.6 | 0.6 | 1.5 | 88.7 | 0 | 0 | 0 | 0 | 0 |
| octa $A_1$ | 18.6 | 14.5 | 8.8 | **14.1** | 18.3 | 0 | 0 | 19.4 | 0 | 3.8 | 2.6 | 94.5 | 0 | 0 | 0 | 0 | 0 |
| octa $A_0$ | 52.2 | 6.2 | 9.2 | **1.4** | 5.6 | 0.6 | 0 | 17.2 | 3.9 | 0.7 | 2.7 | 87.7 | 0 | 0 | 0 | 0 | 0 |
| **Fragmin** | | | | | | | | | | | | | | | | | |
| deca | 58.1 | 4.6 | 5.3 | **6.4** | 4.4 | 0 | 0.9 | 0 | 0 | 0 | 0 | 97.0 | 0 | 0 | 0 | 19.0 | 1.3 |
| deca $A_1$ | 27.0 | 13.8 | 9.0 | **20.3** | 11.6 | 0 | 0 | 0 | 0 | 0 | 0 | 99.5 | 0 | 0 | 0 | 18.2 | 0 |
| deca $A_0$ | 59.8 | 4.1 | 5.1 | **5.0** | 3.3 | 0 | 0 | 0 | 0 | 0 | 0 | 97.4 | 0 | 0 | 0 | 18.7 | 1.5 |
| octa | 56.6 | 2.8 | 4.4 | **10.5** | 2.2 | 0 | 0.9 | 0 | 0 | 0 | 0 | 98.5 | 0 | 0 | 0 | 20.8 | < 1 |
| octa $A_1$ | 34.5 | 4.1 | 10.3 | **21.9** | 7.2 | 0 | 0 | 0 | 0 | 0 | 0 | 99.5 | 0 | 0 | 0 | 21.8 | 0 |
| octa $A_0$ | 58.6 | 2.3 | 2.8 | **8.1** | 2.0 | 0 | 0.8 | 0 | 0 | 0 | 0.8 | 98.8 | 0 | 0 | 0 | 22.6 | 1.0 |

| | $I_{2S}$ | $I - A_{6S}$ | $I - A_{6OH}$ | $G - A_{NS,3S,6S}$ / $[G-(A^*)]$ | $G - A_{NS}$ | $G - A_{NAc}$ | $\Delta U_{2S}$ | $\Delta U$ | $I_{2S}$ red$\alpha$ | $I_{2S}$ red$\beta$ | $G_{2S}$ | $G_{nr}$ | GalA | $U_{redX}$ | Epox |
|---|---|---|---|---|---|---|---|---|---|---|---|---|---|---|---|
| **Clexane** | | | | | | | | | | | | | | | |
| deca | 50.9 | 5.1 | 0.6 | **3.7** | 10.4 | 3.4 | 15.9 | 1.0 | 1.1 | 0.4 | 3.4 | < 1 | 2.5 | 0.7 | <<1 |
| deca $A_1$ | 43.2 | 10.8 | 1.8 | **17.8** | 4.4 | 1.2 | 12.5 | 2.3 | 2.0 | 0.0 | 0.8 | 0.0 | 1.2 | 0.9 | 0.9 |
| deca $A_0$ | 50.0 | 4.4 | 1.0 | **2.3** | 12.5 | 3.5 | 16.3 | 0.8 | 1.8 | 0.4 | 3.4 | 1.4 | 1.4 | 0.9 | <<1 |
| octa | 51.5 | 3.8 | 0 | **3.7** | 8.7 | 0.9 | 22.2 | 1.2 | 2.1 | 0.9 | 3.0 | 1.5 | 1.5 | 0 | 0 |
| octa $A_1$ | 39.4 | 8.4 | 0 | **23.5** | 0 | 0 | 16.9 | 1.8 | 0 | 0 | 0 | 0 | 0 | 0 | 0 |
| octa $A_0$ | 50.8 | 2.9 | 0 | **2.1** | 10.7 | 1.1 | 23.8 | 0.8 | 1.7 | 1.2 | 3.7 | 1.6 | 0.6 | 0 | 0 |
| **Innohep** | | | | | | | | | | | | | | | |
| deca | 59.8 | 5.6 | 1.5 | **2.0** | 9.9 | 2.5 | 18.0 | 0 | 0 | 0 | 0 | 0 | 0 | 0 | 0 |
| deca $A_1$ | 46.3 | 17 | 0 | **12.7** | 5.3 | 0 | 18.8 | 0 | 0 | 0 | 0 | 0 | 0 | 0 | 0 |
| deca $A_0$ | 58.2 | 5.7 | 1.6 | **1.0** | 11.1 | 2.7 | 18.7 | 0 | 0 | 0 | 0 | 0 | 0 | 0 | 0 |
| octa | 60.5 | 5.0 | 0.4 | **2.1** | 9.6 | 1.1 | 20.6 | 0 | 0 | 0 | 0 | 1.7 | 0 | 0 | 0 |
| octa $A_1$ | 45.5 | 13.1 | 0 | **15.6** | 2.5 | 0 | 23.3 | 0 | 0 | 0 | 0 | 0 | 0 | 0 | 0 |
| octa $A_0$ | 60.1 | 5.2 | 0.9 | **1.2** | 9.6 | 1.2 | 20.6 | 0 | 0.4 | 0 | 0 | 1.5 | 0 | 0 | 0 |
| **Fragmin** | | | | | | | | | | | | | | | |
| deca | 81.6 | 4.7 | 0.7 | **5.8** | 4.8 | 1.5 | 0 | 0 | 0 | 0 | 0 | 1.5 | 0.9 | 0 | 0 |
| deca $A_1$ | 66.0 | 14.2 | 0 | **17.5** | 2.3 | 0 | 0 | 0 | 0 | 0 | 0 | 0 | 0 | 0 | 0 |
| deca $A_0$ | 81.8 | 3.9 | 0.6 | **5.0** | 5.1 | 2.1 | 0 | 0 | 0 | 0 | 0 | 2.9 | 1.4 | 0 | 0 |
| octa | 84.7 | 2.7 | 0 | **8.2** | 2.9 | 0.6 | 0 | 0 | 0 | 0 | 0 | 4.6 | 0.9 | 0 | 0 |
| octa $A_1$ | 69.7 | 3.2 | 0 | **22.6** | 4.4 | 0 | 0 | 0 | 0 | 0 | 0 | 0.0 | 0 | 0 | 0 |
| octa $A_0$ | 88.2 | 2.5 | 0 | **6.1** | 2.4 | 0 | 0 | 0 | 0 | 0 | 0 | 4.2 | 0.7 | 0 | 0 |

Table 4: Quantitative evaluation (mol %) of HSQC-NMR spectra of high affinity ($A_1$) and no affinity ($A_0$) octasaccharides and decasaccharides of Clexane, Innohep and Fragmin. In the upper panel hexosamin monomer and dimers are reported; in the lower panel uronic acid monomers and dimers are shown.

In the different oligosaccharidic fractions the observed GA* % contents can be correlated with preliminary data of their anti factor-Xa specific activity (aXa), of the different oligosaccharides. The $A_{1,decasaccharidic}$ fraction of Clexane, Innohep, and Fragmin showed anti factor-Xa specific activity of 150, 250 and 310 U/mg respectively, while the $A_{0,decasaccharidic}$ fractions displayed very low specific activity of 10, 5 and 30 U/mg respectively. The same trend was observed for the corresponding octasaccharides of $A_1$ fraction endowed of 280, 120 and 300 U/mg, whereas all the corresponding $A_{0, octa}$ were found almost inactive (lower than 10 U/mg).

This is effectively illustrated by comparison of RP- IP HPLC/MS profiles of $A_0$ and $A_1$ octasaccharide sub-fractions of Clexane (fig. 7b) . The mass of the major peak of the $A_1$ fraction corresponds to an unsaturated octasaccharide bearing a 4, 5 unsaturated uronic acid at the non reducing end, ten sulfate and one acetyl groups ($\Delta 8$, 10, 1, ) to which, based on knowledge consolidated in studies on AGA*IA- containing oligosaccharides,[8] the prevalent structure $\Delta$U2S-GlcNS, 6S- IdoA- GlcNAc, 6S- GlcA- GlcNS, 3, 6, S- IdoA2S- GlcNS, 6S (OCTA- 3 in the *reference 8*) can be assigned. Species contained in the $A_1$ fraction of Enoxaparin are mainly unsaturated octasaccharides ($\Delta 8$) which differ from each other only for 1- 2 sulfate groups per molecule. Since the molar percent of the marker G- A* determined by NMR in this fraction (23.5/2 = 11.75; see table 4) is only slightly lower than the theoretical value (12.5) calculated for the structure of ATBR containing octasaccharides, this is a proof- of- principle that the HPLC/MS profiles of LMWH sub- fractions with affinity for AT (An) coupled with NMR quantitative data provide a biologically relevant approach to structural characterization of LMWHs.

Data reported in table 4 relative to Fragmin $A_1$ octasaccharide fraction, permit to identify an undescribed variant of the ATBR sequence. In fact, NMR data show the significantly reduced percentage of the expected IdoA content and also a reduced amount of GlcNAc residue in the $A_1$ octasaccharide fraction. As a consequence, being absent the signal of non- reducing end glucuronic acid, the ATBR sequence of these $A_1$ fractions contains the- $I_{2S}$- $A_{NS,6S}$- G- $A_{NS,3S,6S}$- $I_{2S}$- $A_{NS,6S}$- structural variant where IdoA$_{2S}$ instead of IdoA and $A_{NS,6S}$ instead of $A_{NAc,6S}$ are present before AGA*IA sequence The observation is compatible with HPLC- MS profile. The lack of IdoA is well evident in the elaboration of the NMR results as reported in table 5, where for Fragmin and Clexane $A_1$ oligomers the contribution of each uronic residue of the I- AGA*IA sequence are subtracted to the corresponding ones of table 4, using G- A* values as calibrators. Also the Clexane $A_1$ octasaccharide shows a lack of IdoA residue. Accordingly our data indicate that mATbr sequences can be concentrated by AT affinity chromatography, thus improving the characterization and bioanalytical profiling of LMWHs.

| Uronic acid | $I_{2S}$ | I - A6S + I - A6OH | $G$ - $A_{NS}$ | $G$ - $A_{NAc}$ | $\Delta U_{2S}$ | $\Delta U$ |
|---|---|---|---|---|---|---|
| *SAMPLE* | | | | | | |
| **Clexane** | | | | | | |
| deca HA | **1,4** | **-0,5** | *0,2* | *0,1* | *0,7* | *0,1* |
| octa HA | **0,67** | **-0,64** | *0* | *0* | *0,72* | *0,07* |
| **Fragmin** | | | | | | |
| deca HA | **2,8** | **-0,2** | *0,2* | *0* | *0* | *0* |
| octa HA | **2,2** | **-0.8** | *0,2* | *0* | *0* | *0* |

Table 5 : Elaboration of Clexane and Fragmin data reported in table 4, relative to uronic acids (lower panel), by subtraction of the contribution of the intact ATBR sequence and of the IdoA that are present before the ATBR sequence ($-I-A_{NAc}-G-A_{NS,3,6S}-I_{2S}-A_{NS,6S}-$; bold characters) from A1 deca- and octa-oligosaccharides. Each amount of G-A* % values are used as calibrator to calculate the theoretical amount of ATBR residua that are present in the A1 fractions. Residues not part of ATBR sequence are evaluated as % value respect to G-A* content. Negative values indicate a lack of an intact ATBR component, suggesting the deficiency of these residues in the oligosaccharide families. Positive values indicate the exciding contribution of a residue respect to the expected contribution of ATBR, that together with residues not part of ATBR sequence (see italic characters) allow to evaluate the environment in which ATBR is located. Shorter is the size of the oligosaccharide family, higher is the information about the structure. In any case, this methodology facilitate the structural differentiation among the different oligomer families.

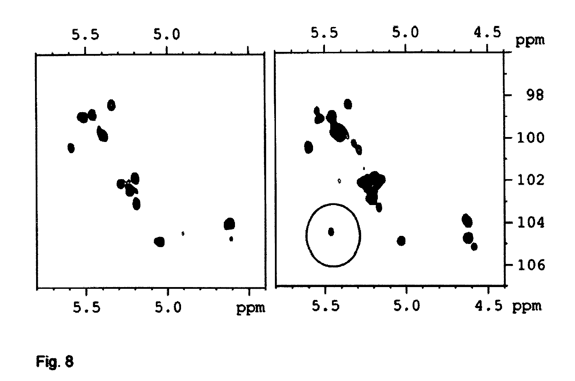

In NMR spectra of Fragmin and some of its fractions (i.e.$A_{0,octa}$ and $A_{0,deca}$ saccharides) unassigned signals have been identified. The assignment of these signals, by homonuclear (1D- TOCSY) and heteronuclear correlation spectroscopy (HSQC and HSQC- TOCSY), was possible on $A_{0,deca}$ saccharides, since they were particularly enriched. Proton and carbon chemical shifts are reported in table 6. We discovered that they correspond to a ring contracted unit derived from a glucosamine residue. Being one of the signals easily detectable and quantifiable in the anomeric region of HSQC spectrum (5.45 ppm/ 104.4 ppm; see $A_x$ label in table 4 and fig. 8), it can be regarded as a marker of the peculiar depolymerizing process of Fragmin. The evaluation of such a signal became a method to identify univocally the brand process of Fragmin and it is applicable both to Fragmin and its fractions. Signals corresponding to ring contracted unit derived from glucosamine residue of Fragmin were observed only in the $A_0$ but not in the $A_1$ decasaccharide fractions (*Fig 8*) .

Table 6 Chemical shifts of $^1$H and $^{13}$C signals assigned to the ring contracted unit derived from glucosamine residue of $A_{0,deca}$ of Fragmin.

|  | H1/C1 | H2/C2 | H2'$_{a/b}$/C2' | H3/C3 | H4/C4 |
|---|---|---|---|---|---|
| 1H | 5.45 | 2.53 | 3.90-3.85 | 4.53 | 4.45 |
| 13C | 104.4 | 52.3 | 59.1 | 79.9 | nd |

Heparin and LMWH constituents obtained through the affinity chromatography followed by molecular weight fractionation are further characterized by physico- chemical methods, including NMR spectroscopy and mass spectrometry, aided if necessary by chemical and or enzymatic treatments. More in details, structures are identified directly from mass/ charge values in their LC/MS chromatograms (i.e. RP- IP- HPLC/MS, SAX- HPLC/MS) where the presence of double bond is detected by an on line UV detector. When needed for refined assignments, the molecular size and structure of isolated components obtained from the different homogeneous size samples is determined using MS techniques such as MS/MS and/or with a complementary approach based on cleavage of the LMWHs (or their fractions) with heparinases (heparin lyase I, II, or III, or their mixture) . Furthermore, glycol- splitting (permitting to resolve "isomeric" oligosaccharides) and Smith degradation (permitting to profile LMWHs also as a function of position and density of 2-O- sulfated uronic acid residues along the heparin chains) can optionally be used as complementary tools for structural analysis of $A_{0/n}$ and/or $A_{0/n}B_m$ oligosaccharides. Structural assignments are investigated and or confirmed by quali- and/or quantitative mono- and bi- dimensional, homo- and/or heteronuclear NMR analyses.

EXAMPLES

The LMWHs used in the examples are brand products purchased on the market while some LMWH active principles, such as Enoxaparin a-d, were prepared in laboratory on a pilot scale according to described process of the originator.
Lovenox and Clexane are brands of a LMWH produced by Sanofi Aventis via chemical beta elimination process, their active principle is enoxaparin. Innohep is a brand of Leo Pharma produced by enzymatic beta elimination, its active principle is tinzaparin.
Fragmin is a brand of Pfizer (active principle Dalteparin) and Fraxiparin is a brand of Glaxo SmithKline (active principle Nadroparin), both produced by nitrous acid depolymerisation. Fluxum is a brand of Alfa Wassermann obtained through radical depolymerisation (active principle Parnaparin). Each LMWH brands are used as reference standard for their corresponding active principle ingredients (API). To identify the range values of each brand structural elements, a preferred approach is based on databases generated by analysing different batches of each LMWH brand.
The characterization of mono- and di- saccharidic components is performed a) on intact products by monodimensional $^1$H and $^{13}$C NMR and/or heteronuclear bidimensional experiments, described in literature[24,25,26,27,28,29] and/or b) on exhaustively chemically or enzymatically depolymerized products by chromatography through but not exclusively SAX- HPLC- UV/MS methods.
The determination of molecular weight is performed by every adequate technique. In a preferred embodiment the determination is performed through a chromatographic approach using a detector array such as but not exclusively light scattering associated with viscometer and refractive index, avoiding the use of any chromatographic calibrator.

. Example 1a:

an analytical AT-Sepharose column of 5 ml was prepared by linking antithrombin (AT) to CNBr-activated Sepharose 4B according to literature[13].
The maximum heparin binding capacity of the AT-Sepharose column was assessed by loading different amounts of pig mucosal unfractionated heparin (UFH) in the range 1-3 mg and re-loading each corresponding $A_0$ fraction until the $A_1$ fraction no longer appears.
A sample of 1.5 mg of each LMWH, dissolved in 1.5 ml of Tris-HCl 0.05 M, pH 7.4, 0.05 M NaCl, was loaded onto AT-Sepharose column first with 15 ml of the same buffer to recover the no affinity ($A_0$) fraction, then NaCl concentration was increased from 0.05 M to 2.5 M in 25 ml of Tris-HCl 0.05 M, pH 7.4 buffer, to recover the affinity fraction ($A_1$) making a linear gradient elution. Effluent fractions of about 1.5 ml were analysed for uronic acid content by carbazole reaction[31].
The relative content of the $A_1$ fraction, evaluated as weight % in different LMWH samples, is reported in table 1a. A typical gradient elution profile of a LMWH is presented in fig. 2a.

. Example 1b:

A sample of 1.5 mg of each LMWH, dissolved in 1.5 ml of Tris-HCl 0.05 M pH 7.4 0.05 M NaCl, was loaded onto the 5 ml analytical AT-Sepharose column prepared in and eluted first with 15 ml of the same buffer to recover no affinity ($A_0$) fraction, then with 15 ml of Tris-HCl 0.05 M pH 7.4, 2.5 M NaCl, to recover the affinity ($A_1$) fraction. The relative content of the $A_1$ fraction, evaluated as weight % in different LMWH samples, is reported in table 1a. Effluent fractions of about 1.5 ml were analysed as described in example 1a.

. Example 1c:

a preparative AT-Sepharose column of 180 ml was prepared and tested for its capacity as previously described in example 1a.
A sample of 70 mg of each LMWH, dissolved in 7 ml of Tris-HCl 0.05 M, pH 7.4, 0.05 M NaCl, was loaded onto an AT-Sepharose column and eluted first with 360 ml of the same buffer to recover no affinity ($A_0$) fraction, then with 540 ml of Tris-HCl 0.05 M pH 7.4, 2.5 M NaCl, to recover the affinity ($A_1$) fraction. Effluent fractions of about 30 ml were analysed as described in example 1a. A typical elution profile of a LMWH is presented in fig. 2b.

. Example 1d:

A sample of 70 mg of Lovenox or Enoxaparin, dissolved in 7 ml of Tris-HCl 0.05 M, pH 7.4, 0.05 M NaCl, was loaded onto a preparative AT-Sepharose column (180 ml) and eluted first with 360 ml of the same buffer to recover no affinity ($A_0$) fraction, then with 360 ml of Tris-HCl 0.05 M pH 7.4, 1.0 M NaCl, to recover low affinity ($A_1$) fraction and last with 360 ml of Tris-HCl 0.05 M pH 7.4, 2.5 M NaCl, to recover high affinity ($A_2$) fraction. Under the described experimental conditions, the $A_1$ fraction contains the lowest affinity component. The relative content of the $A_1$ and $A_2$ fractions, evaluated as weight % by carbazole assay[31] in three different Lovenox batches, are reported in table 1b. A typical elution profile of $A_1$ Enoxaparin is presented in fig. 2c.

. Example 2:

the $A_0$, $A_1$, $A_2$ fractions, obtained as previously described in examples 1c and 1d, were desalted through a three-step procedure as follows:[21]
most of the salt was removed by an ultra-filtration step by using an Amicon stirred cell (Millipore, model 8200) equipped with a cellulose acetate membrane having a molecular weight cut-off of 500 Da. Solution volumes (about 200 ml) containing $A_0$ or An fractions of each LMWH were concentrated to 100 ml by ultra-filtration, under a constant pressure of 40 psi, and added of 100 ml of deionized water. The operation was repeated twice for the $A_0$ and $A_1$ fraction and three times for the $A_2$ fractions. Then the solutions were finally concentrated to 5 ml;
5 ml of partially desalted $A_0$, $A_1$ or $A_2$ fraction were loaded on TSK-gel HW40S column (2.6 x 58 cm) and eluted with 10% ethanol in water at a flow-rate of 4.8 ml/min. Effluent fractions of about 6 ml were analysed for their absorbance at 210 nm; desalting was finally refined by stripping traces of Tris salt by a cation-exchange chromatography. Fractions corresponding to the peak of each desalted oligosaccharide were mixed together and applied to an Amberlite IR-120 [$H^+$] column (2 x 12.5 cm). The solution eluting from the column was recovered and neutralised with 0.1 N NaOH and freeze dried.

. Example 3:

the RP IP HPLC- MS analytical profile of $A_0$ and $A_1$ fractions of Clexane and Fragmin isolated as in example 1c and $A_1$ fraction of Enoxaparin isolated as in example 1d, were determined using a LC system (Dionex Ultimate 3000, Dionex) equipped with degasing system (model LPG- 3400), pump (model LPG- 3400A), autosampler (model WPS-3000TSL) and UV- detector (model VWD- 3100) and coupled with ESI- QTOF mass- spectrometer (micrOTOFQ, Bruker Daltonics) as a detector.
HPLC analyses of oligosaccharides were carried out using a Kinetex C18 analytical column (100 x 2.1 mm I.D., 2.6 μm particle size, Phenomenex) with Security Guard Cartridges Gemini C18 (4 x 2.0 mm, Phenomenex) . The mobile phases A and B containing 10 mM DBA and 10 mM $CH_3COOH$ in water- methanol = 90: 10 (v/v) and 100% methanol, respectively, were delivered at 0.1 ml/min, pH value of phase A and B were 6.2 and 7.9, respectively (fig.s 3b and 3c) . Different gradients were used in function of oligosaccharide compositions. To obtain the characteristic LC- MS profiles of LMWH or their affinity fractions the follow gradient was used: 0 min- 20% B, 15 min- 20 % B, 55 min- 40%B, 100 min- 50%B, 150 min- 70%, 170 min- 90% B, 180 min- 90% B, 182 min- 20% B, 200 min- 20% B (fig. 3b and c) . For separating oligosaccharides with higher molecular weights components the LC gradient was optimized as follows: 0 min- 20% B, 5 min- 25 % B, 40 min- 40% B, 50 min- 40%B, 80 min- 45%, 90 min- 45% B, 130 min- 60% B, 140 min- 60% B, 190 min- 90% B, 200 min- 90% B, 202 min- 20% B, 220 min- 20% B (fig 3a) . The three last points of each gradient were used for washing and equilibrating of the chromatographic column before the injection of the next sample.

The injection volume of each sample was $7 \div 5$ $\mu$l of a solution containing $4 \div 2$mg/ml of LMWHs or their fractions.

The MS conditions were as follows: ESI was in negative ion mode, drying gas temperature was +180 °C, drying gas flow- rate was 7 ml/min, nebulizer pressure was 0.9 bar; capillary voltage was +3.2 kV. The mass spectra of the oligosaccharides were acquired in a scan mode (m/z scan range 200- 3000) .

The MS spectrum of each single peak permits the determination of the mass of the component (s) . Quantitative evaluations can be obtained by using different appropriate calibration standards.

. Example 4:

RP- IP- HPLC/ESI- QTOF- MS analysis of each LMWHs was performed before the affinity chromatography following the method described in example 3 in order to separate the oligosaccharide components.

Typical TIC profiles of Clexane and Innohep, divided into three mass ranges, are reported in fig.s 6a and 6b.

. Example 5a:

the low pressure SEC chromatography[32] on Biogel P6 was performed to separate and quantitatively evaluate the size homogeneous oligosaccharide components of both LMWHs and their $A_{0/n}$ fractions:

A sample of 100- 150 mg of each different LMWH was dissolved in 2- 5 ml of water and loaded onto a Biogel P6 column set (two glass columns coupled in series, each one of 2.6 x 96 cm) and eluted with $NH_4Cl$ 0.25 M at a flow rate of 1.8 ml/min, collecting fractions of about 17 ml each. Elution profiles were recorded by UV absorbance at 210 nm. The di- tri, tetra, penta, hexa, octa, deca, dodeca, tetradeca and the larger oligosaccharide components were isolated. The fraction amounts were determined by evaluation of UV absorbance at 210 nm.

In such chromatographic conditions, the number of theoretical plates calculated for the tetrasaccharide peak is about 6000. Figure 5 shows a typical chromatographic profile obtained under such a condition of a Lovenox batch where it is possible to identify in addition to the even oligomers also odd sequences. Table 2a reports the average content (weight %) of the size homogeneous oligosaccharidic components of Lovenox, Innohep and Fragmin, obtained from seven, five and six chromatographic separations, respectively. Such data prove the reproducibility of the experiment performed on three LMWH brands. Table 2b reports the average content (weight %) of size homogeneous oligosaccharidic components determined on six different batches of Lovenox. The range of the weight % for each oligosaccharide is reported and compared with data from Patent US, 7, 790, 466 B1 and US  7, 790, 466 B1. A number of additional oligosaccharidic components are separated and quantified with our procedure. The volume containing each oligosaccharide peak, ranging from 100 ml to 250 ml, was desalted as in example 2.

. Example 5b:

A sample of 20-100 mg of each $A_0$ and $A_1$ fractions of Clexane, obtained by pooling corresponding fractions derived from several affinity chromatography experiments, according to 1c, was dissolved in 1-5 ml of water and loaded onto a Biogel P6 column set and the same procedure described in example 5a was applied.

The tri, tetra, penta-hexa, hepta-octa, deca, dodeca, tetradeca-saccharides together with the higher oligosaccharides were isolated.

The same procedure was applied on $A_0$ and $A_1$ fractions of Innohep and Fragmin.

. Example 6:

LC- MS analysis of $A_0B_m$ and $A_1B_m$ oligosaccharides fractioned by affinity chromatography and SEC was performed on an Agilent 1100 HPLC system (Agilent Technologies) coupled to an Esquire 3000 Plus (Bruker, Germany) as mass detector. Reversed- phase separation of LMWH fractions was carried out on a Phenomenex Kinetex®- C18 column (2.1 x 100 mm, 2.6 $\mu$m, 100 A) . 7 $\mu$l of a solution containing 4mg/ml of $A_1$octa oligosaccharides of Clexane are injected onto the column. The column was held at 80% Solvent A (20 mM DBA in water) and 20% Solvent B (20 mM DBA in methanol) for 20 min followed by a 10 min gradient from 20% B to 40% B. The column was held at 40% B for 10 min. Subsequently, a 20 min. gradient from 40% B to 90% B was applied and once 90% B was reached, this was kept for 10 min. Column conditioning for subsequent injections was established by a 5 min. gradient from 90 % B to 20 % B and a 30 min. equilibration at this solvent mixture. The flow rate and column temperature were maintained at 0.15 mL/min and 25 °C, respectively, throughout the run. Mass spectrometric analysis were performed on an Esquire 3000 Plus electrospray ion trap (Bruker Daltonics, Bremen, Germany) . Acquisition parameters for ESI- ion trap mass spectrometer were set up as following: negative polarity, mass range 300- 1000 m/z, capillary +3166V, nebulizer gas 60.0 psi, drying gas flow 12.0 L/min, drying gas temperature 350°C.

The $A_1$octa and $A_0$octa of Fragmin, the $A_1$octa, $A_1$deca, $A_0$octa and $A_0$deca of Innohep and Clexane oligosaccharides are analyzed according to the same conditions.

Fig.s 7a and 7b report the LC- MS profiles of $A_1$octa- $A_1$deca and $A_0$octa- $A_0$deca oligomers of Clexane and Innohep, respectively. Fig. 7c reports the LC- MS profiles of $A_1$ octa and $A_0$ octa of Fragmin. The MS spectrum of each single peak permits the determination of the mass values of the component (s) and the assignment of each peak of the profile is reported. Quantitative evaluations could be done using different appropriate calibration standards.

. Example 7:

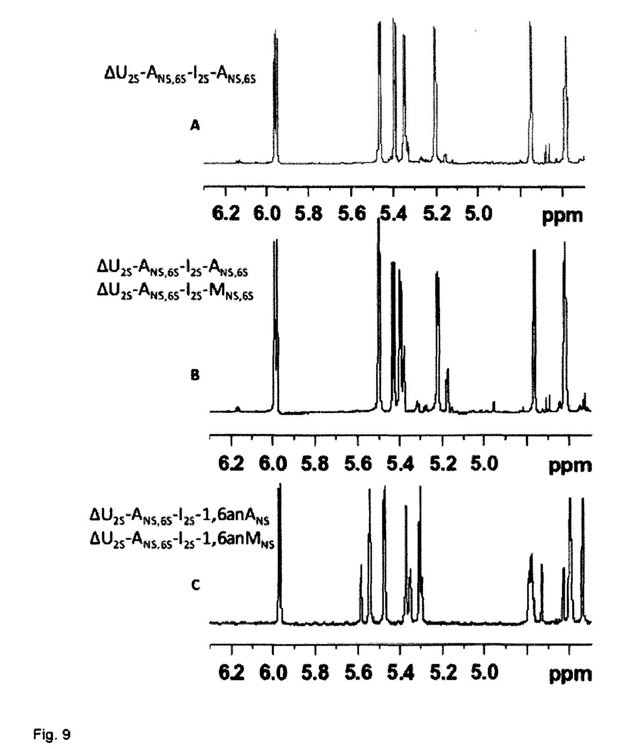

fractionations of $A_0$ and $A_1$ tetrasaccharide components of Lovenox and Innohep in order to to isolate single tetrasaccharidic components were achieved by preparative RP-IP-HPLC by using a LC system (KNA5316, Knauer) equipped with degasing system, autosampler (model HTA) and UV-detector (model UVIS-200, Lab Service) and using a Eurospher II C18 column (250 x 8 mm, Knauer). The mobile phases A and B containing 10 mM DBA and 10 mM $CH_3COOH$ in water-methanol = 90:10 (v/v) and 100% methanol, respectively, were delivered at 2 ml/min, pH value of phase A and B were 6.2 and 7.9, respectively, according to a multi step gradient. The injection volume of each sample was $100 \div 120 \ \mu l$ of a solution containing $4 \div 5$mg/ml of $A_0$ and $A_1$ tetrasaccharide components. The elution profiles were determined by evaluating of UV absorbance at 210 nm. Fractions of about 0.5 ml were collected. 26 fractions obtained by pooling corresponding fractions derived from several preparative RP-IP-HPLC separations, were analysed by RP-IP-HPLC/MS as described in Example 3 and by $^1$H and heteronuclear bidimensional NMR spectroscopy (HSQC-NMR) as described in Example 8. The $^1$H-NMR spectra of some tetrasaccharides isolated from the whole $A_0$ tetrasaccharidic component are reported in Figs 9a, 9b and 9c.

. Example 8:

heteronuclear bidimensional NMR characterization (HSQC-NMR) was performed on oligomeric size fractions isolated as described in example 5b.

Table 4 reports the quantitative distribution of monomer-dimer components divided in hexosamine (upper panel) and uronic acid (lower panel) for the $A_0$ and $A_1$ octa and decasaccharidic fractions.

Among the signals of minor components, we detected in the anomeric region of the HSQC spectrum of Fragmin a signal at 5.45 ppm/104.4 ppm, that can be attributed to a ring contracted unit derived from glucosamine residue inside the chain (see $A_x$ label in fig. 8).

The assignment of this residue was performed by homonuclear (1 D-TOCSY) and heteronuclear correlation spectroscopy (HSQC and HSQC-TOCSY). Proton and carbon chemical shifts are reported in table 6. Signals corresponding to ring contracted glucosamine  residue of dalteparin were observed only in the $A_0B_4$ but not in the $A_1B_4$ decasaccharide fractions of dalteparin (fig. 8).

. Example 9:

Glycol- split heparins (gs- heparins) and LMW heparins were prepared by exhaustive periodate oxidation and borohydride reduction. About 50 mg of each heparin sample were dissolved in 1.5 ml of $H_2O$, and 1.5 ml of 0.2 M $NaIO_4$ were added to the solutions. The solutions were stirred at 4°C overnight in the dark. The reactions were stopped by adding 150 $\mu l$ of ethylene glycol maintaining the agitation for a further hour at 4°C. Solid sodium borohydride (32 mg) was added to the retentate solutions under stirring at room temperate. After 3 h the pH was adjusted to 4 with 1 M HCl, and then acidic solutions were neutralized with 1 M NaOH. The gs- heparin were desalted with TSK column, HW 40s resin [33];

the substrate (2- 3 mg) was dissolved in 1: 1 (v/v) mixture of 100 mM sodium acetate buffer (pH 7.0) and 10 mM calcium acetate to obtain a solution with concentration of 7.7 mg/ml. To carry out the enzymatic cleavage reaction 13 $\mu l$ of each sample were diluted by 144 $\mu l$ of 1: 1 (v/v) mixture of 100 mM sodium acetate and 10 mM calcium acetate, and then 3 $\mu l$ of heparin lyases mixture (1 $\mu l$ of each 2 mU/ $\mu l$ enzyme solution) were added to the heparin solution.

The reaction was stirred at 37°C (Termo shaker TS- 100 Biosan) for 24 h. The reaction was stopped by adding 3 $\mu l$ of 3% HCOOH. Each sample was diluted in water and was analyzed by IP- HPLC/ESI- TOF method. Fig. 4 reports data relative to Innohep and Clexane. The different relative proportions of families of glycol- split (gs) tetra and pentasulfated tetrasaccharides, and hexa- and hepta- sulfated hexasaccharides depend on the type of LMWH. Some of the structures (i.e.: $\Delta4, 6, 0$+gs, $\Delta6, 7, 1$+2gs) are components derived from the ATBR sequence and can be taken as markers of it.

The analysis was performed on a HPLC- MS system equipped with degasing system, gradient pump, autosampler, and coupled with ESI- QTOF mass- spectrometer (micrOTOFQ, Bruker Daltonics) and UV- detectors.

The follow gradient was used: 0 min- 17% B, 10 min- 17 % B, 30 min- 42%B, 50 min- 50%B, 65 min- 90% B, 75 min- 90% B, 76 min- 17% B, 95 min- 17% B. The injection volume of each sample was 5 $\mu$l (2mg/ml) . The MS conditions were as follows: ESI was in negative ion mode, drying gas temperature was +180 °C, drying gas flow- rate was 7 ml/min, nebulizer pressure was 0.9 bar; capillary voltage was +3.2 kV. The mass spectra of the oligosaccharides were acquired in a scan mode (m/z scan range 200- 3000) .

. Example 10:

$A_0$ and $A_1$ octa and decasaccharidic fractions of Clexane, Innohep and Fragmin (isolated as described in 5b) were characterized by heteronuclear bidimensional NMR (HSQC- NMR) as reported in example 8.

Tables 4a and 4b report the quantitative distribution of monomer- dimer components divided in hexosamine (Table 4 a) and uronic acid (Table 4b) . Data were elaborated by subtracting the contribution of the intact ATBR sequence to facilitate the structural differentiation between the different oligomers.

Table 5 provides information about the prevalent structure of oligosaccharides endowed of affinity for AT by evaluating of the ratio among the different detected residues. Data are obtained from Table 4 by the following scheme of calculation referred as an example to $A_1$ Clexane octasaccharide:

a) Reference area GA* = 23.5

b) Considering I- $A_{NAc,6S}$- G- $A_{NS,3S,6S}$- $I_{2S}$- $A_{NS,6S}$ (IAGA*IA) the expected regular main hexasaccharidic sequence endowed of affinity for AT, the ratio of each of its residues respect to GA* is calculated:

$$ I = \frac{8.4}{23.5} = 0.36; \quad ; \quad G - A* = \frac{23.5}{23.5} = 1; \quad A* = \frac{23.3}{23.5} = 0.99; \, I2S = \frac{39.4}{23.5} = 1.68; $$

$$ ANAc - G = \frac{8.4}{23.5} = 0.36 $$

c) To evaluate the real contribution of such residues to the binding structure, 1 (GA*) was subtracted to the number obtained in point b with respect to the hypothesized structure with affinity. Positive and negative numbers indicate exceeding or deficiency of residues respectively.

$$ I = (0.36 - 1) = -\mathbf{0.64}; \qquad G = (1 - 1) = \mathbf{0}; \qquad A* = (0.99 - 1) = -\mathbf{0.01} $$

$$ I2S = (1.68 - 1) = \mathbf{0.68}; \quad ANAc - G = (0.36 - 1) = -\mathbf{0.64}; $$

d) To evaluate the relative amount of residues detected but not included in IAGA*IA sequence, their ratio respect to GA* is calculated (see italic characters in Tab.5):

$$ I2S = \frac{45.5}{23.5} = \mathbf{1.94}; \quad ANS - G = \frac{0}{23.5} = \mathbf{0}; \quad \Delta U2S = \frac{16.8}{23.5} = \mathbf{0.7}; \quad \Delta U = \frac{1.8}{23.5} = \mathbf{0.08}; $$

$$ ANS - G = \frac{13.9}{23.5} = 0.59; \, Gnr = \frac{0}{23.5} = \mathbf{0} = (16.9/) = -\mathbf{0.01} $$

e) As results the average structure is obtained through compensation of non sulfated and sulfated residues (I= -0.64 versus $I_{2S}$= 0.68: $A_{NAc}$-G =-0.64 versus $A_{NS}$-G =0.59) and through the content of non reducing end group ($\Delta U_{2S}$=0.70; $G_{nr}$= 0). The 59 - 64% of the structure of this fraction is:

$$ \Delta U_{2S}\text{-}A_{NS,6x}\ I_{2S}\text{-}A_{NS,6x}\text{-}G\text{-}A_{NS,3S,6S}\text{-}I_{2S}\text{-}A_{NS,6x} $$

x=SorOH

Where the uronic acid preceding AGA*IA sequence is a 2-O sulfated iduronic acid instead of nonsulfated iduronic acid and the following unit is a N-sulfated glucosamine instead of a N-acetyl one.

References:

1. B. Casu, U. Lindahl. Structure and biological interactions of heparin and heparan sulfate. Advances Carbohydr. Chem. Biochem. 57, (2001) 159-206.

2. R.J. Linhardt. Heparin: structure and activity. [2003 Claude S. Hudson Award address in carbohydrate chemistry], J. Med. Chem. (2003) 2551-2564.

3. B. Casu, Structure and active domains of heparin. In: H.G. Garg, R.J. Linhardt, C.A. Hales (ed.s) Chemistry and Biology of Heparin and Heparan Sulfate, Elsevier (2005) Amsterdam, pp. 1-28.

4. H.E. Conrad, Heparin binding proteins.( 2003) Academic Press, San Diego, CA.

5. I Capila, R.J. Linhardt. Heparin-protein interactions. Angew. Chem. Int. Ed. 41 (2002) 390-412.

6. J.M. Whitelock, R.V. Iozzo. Heparan sulfate: a complex polymer charged with biological activity. Chem. Rev. 105 (2005) 2745-2764.

7. J.R. Bishop, M. Schuksk, J.D. Esko, Heparan sulfate proteoglycans fine-tune mammalian physiology. Nature 446 (2007) 1030-1037.

8. M. Guerrini, S. Guglieri, B. Casu, G. Torri, P. Mourier, C. Boudier, C. Viskov. Antithrombin-binding octasaccharides and role of extensions of the active pentasaccharide sequence in the specificity and strength of interaction. Evidence for very high affinity induced by an unusual glucuronic acid residue. J Biol Chem. 26: (2008) 26662-26675.

9. M. Guerrini, S. Elli, D. Gaudesi, G. Torri, B. Casu, P. Mourier, F. Herman, C. Boudier, M. Lorenz and C. Viskov. Effects on molecular conformation and anticoagulant activities of 1,6-anhydrosugars at the reducing terminal of antithrombin-binding octasaccharides isolated from low-molecular-weight heparin enoxaparin. Med. Chem. 2010 DOI: 10.1021/jm100771s.

10. M. Guerrini, S. Guglieri, D. Beccati, G. Torri, C. Viskov, P. Mourier. Conformational transitions induced in heparin oligosaccharides by binding with antithrombin III. Biochem. J., 15, (2006) 191- 198.

11. A. Bisio, A. Mantegazza, E. Urso, A. Naggi, G. Torri, C. Viskov, B. Casu. High-performance liquid chromatographic/mass spectrometric studies on the susceptibility of heparin species to cleavage by heparanase. Semin. Thromb. Hemost. 33 (2007) 488-495.

12. M. Petitou, C. A. van Boeckel. A Synthetic Antithrombin III Binding Pentasaccharide Is Now a Drug! What Comes Next? Angew. Chem. Int. Ed., 43, 24 (2004) 3118-3133.

13. Höök M, Björk I, Hopwood J et al. Anticoagulant activity of heparin: separation of high-activity and low-activity heparin species by affinity chromatography on immobilized antithrombin. FEBS Lett. 66 (1976) 90-93.

14. I. Capila, N.S. Gunay, Z. Shriver, G. Venkataraman. Methods for structural analysis of heparin and heparan sulfate. In: Chemistry and biology of heparin and heparan sulfate (Garg, H.G., Linhardt, R.J., Hales, C.A., Eds), Elsevier (2005) pp. 55-78.

15. B. Casu, P. Oreste, G. Torri, G. Zoppetti, J.Choay, J.C. Lormeau, M. Petitou, P. Sinaÿ. The structure of heparin oligosaccharide fragments with high anti-(factor Xa) activity containing the minimal antithrombin III-binding sequence. Chemical and 13C-NMR studies. Biochem. J. 197 (1981) 599-609.

16. M. Sundaram, Y. Qi, Z. Shriver, D. Liu, G. Zhao, G. Venkataraman, R. Langer, R. Sasisekharan. Rational design of low-molecular weight heparins with improved in vivo activity. Proc. Natl Acad. Sci. USA 100, (2003) 651-656.

17. R.M. Marks, H. Lu, R. Sundaresan, T. Toida, A. Suzuki, T. Imanari, M.J. Hernáiz, and R.J. Linhardt. Probing the interaction of dengue virus envelope protein with heparin: assessment of glycosaminoglycan-derived Inhibitors. J. Med. Chem. 44 (2001) 2178-2187.

18. M. Guerrini, S. Guglieri, A. Naggi, R. Sasisekharan, G. Torri. Low molecular weight heparins: structural differentiation by bidimensional nuclear magnetic resonance spectroscopy. Semin. Thromb. Hemost. 33 (2007) 478-487.

19. P.A.J. Mourier, C. Viskov. Chromatographic analysis and sequencing approach of heparin oligosaccharides using cetyltrimethylammonium dynamically coated stationary phases. Anal. Biochem. 332 (2004) 299-313.

20. M. Kusche, G. Torri, B. Casu, U. Lindahl. Biosynthesis of heparin. Availability of glucosaminyl- 3- O- sulfation sites. J. Biol. Chem. 265 (1990) 7292- 7300.

21. A. Bisio, D. Vecchietti, L. Citterio, M. Guerrini, R. Raman, S. Bertini, G. Eisele, A. Naggi, R. Sasisekharan, G. Torri. Structural features of low molecular weight heparins affecting their affinity to antithrombin. Thromb. Haemost. 102: 865-873 (2009).

22. E. Vismara, M. Pierini, S. Guglieri, L. Liverani, G. Mascellani, G. Torri. Structural modification induced in heparin by a Fenton-type depolymerization process. Semin. Thromb. Hemost. 33, (2007) 466-477.

23. B. Casu, M. Guerrini, A. Naggi, M. Perez, G. Torri, D. Ribatti, P. Carminati, G. Giannini, S. Penco, C. Pisano, M. Belleri, M. Rusnati, M. Presta. Short heparin sequences spaced by glycol-split uronate residues are antagonists of fibroblast growth factor 2 and angiogenesis inhibitors. Biochemistry 41 (2002) 10519-10528.

24. B. Casu, G. Torri. Structural characterization of low molecular weight heparins. Semin. Thromb. Hemost. 25 (1999) 17-25.

25. M. Guerrini, A. Bisio, and G. Torri. Combined quantitative 1H and 13C nuclear magnetic resonance spectroscopy for characterization of heparin preparations. Semin. Thromb. Hemost. 27 (2001) 473-482.

26. A. Bisio, S. Guglieri, M. Frigerio, G. Torri, E. Vismara, U. Cornelli, D. Bensi, S. Gonella, L. De Ambrosi. Controlled γ-ray irradiation of heparin generates oligosaccharides enriched in highly sulfated sequences. Carbohydr. Polym. 55 (2004) 101-112.

27. M. Guerrini, A. Naggi, S. Guglieri, R. Santarsiero, G. Torri. Complex glycosaminoglycans: profiling substitution patterns by two-dimensional nuclear magnetic resonance spectroscopy. Anal. Biochem. 337 (2005) 35-47.

28. G. Mascellani, M. Guerrini, G. Torri, L. Liverani, F. Spelta, P. Bianchini. Characterization of di- and monosulfated, unsaturated heparin disaccharides with terminal N- sulfated 1, 6- anhydro- β- D- glucosamine or N- sulfated 1, 6-anhydro- β- D- mannosamine residues. Carbohydr. Res. 342 (2007) 835- 842.

29. M. Hricovini, M. Guerrini, G. Torri, S. Piani, F. Ungarelli. Conformational analysis of heparin epoxide in aqueous solution. An NMR relaxation study. Carbohydr. Res. 277 (1995) 11-23.

30. S. Bertini, A. Bisio, G. Torri, D. Bensi, M. Terbojevich. Molecular weight determination of heparin and dermatan sulfate by size exclusion chromatography with a triple detector array. Biomacromol. 6 (2005) 168-173.

31. T. Bitter, H.M. Muir. Quantitative determination of uronic acids with m-hydroxydiphenyl. Anal. Biochem. 4 (1962) 330-334.

32. M.A. Skidmore, J.E. Turnbull. Separation and sequencing of heparin and heparin sulphate saccharides. In: Garg HG, Linhardt RJ, Hales CA eds. Chemistry and Biology of Heparin and Heparan Sulfate. Elsevier Ltd; Ch. 6 (2006) 179-201.

33. A. Naggi, B. Casu, M. Perez, G. Torri, G. Cassinelli, S. Penco, C. Pisano, G. Giannini, R. Ishai-Michaeli, I.

Vlodavsky.Modulation of the heparanase-inhibiting activity of heparin through selective desulfation, graded N-acetylation, and glycol-splitting. J. Biol. Chem. 280 (2005) 12130-12113

**Claims**

1. Method for the characterization of heparin and heparin derivatives comprising isolation and quantification of fractions having different affinities for antithrombin (AT) by affinity chromatography on immobilized antithrombin and sub-fractionation of said fractions into size- homogeneous families of oligosaccharides by size- exclusion chromatography (SEC), or into size- and charge- homogeneous families of oligosaccharides by ion pair- reverse phase high performance liquid chromatography (IPRP/ HPLC),
   wherein fractions without affinity are recovered from the affinity column in isocratic conditions using NaCl 0.05M as eluent, whereas fractions with high affinity are eluted using NaCl ranging from 0.5M to 2.5M.

2. Method according to claim 1 wherein SEC fractionation is performed on a column of at least 1.6 m length and 2 to 5 cm of diameter, using low pressure conditions and a number of theoretical plates of at least 6000.

3. Method according to claims 1-2 further comprising sub-fractionation of fractions having different affinities for antithrombin into charge- homogeneous families of oligosaccharides by ion-exchange chromatography.

4. Method according to claims 1-3, where the number of fractions obtained by affinity chromatography are comprised between 2 and 6, preferably between 2 and 3.

5. Method according to claims 1-4, comprising physico-chemical characterization of isolated fractions by one or more of the following: NMR spectroscopy, IPRP chromatography on line or off line with MS spectroscopy, MALDI-TOF spectrometry, or any kind of MS/MS and/or FT-MS spectrometry.

6. Method according to claims 1-5, comprising HPLC/MS analysis of the glycol-split derivatives of heparin, heparin derivatives and chromatographic fractions, wherein said glycol-split derivatives are obtained by periodate oxidation followed by borohydride reduction.

7. Method according to claims 1-6, where heparin and heparin derivatives are selected from low-molecular weight heparins (LMWH) and unfractionated heparins (UFH).

8. Method according to claim 7, where LMWHs are selected from Enoxaparin, Tinzaparin and Dalteparin.

9. Method for validation of fragmin samples by homonuclear (1D-TOCSY) and heteronuclear correlation spectroscopy (HSQC and HSQC-TOCSY) of fragmin as such or of fractions of fragmin without affinity for antithrombin, wherein a signal is identified in the anomeric region of HSQC spectrum having a chemical shift of 5.45 ppm/104.4 ppm.

10. Use of the method according to claim 9 for the evaluation of LMWHs manufacturing process, comparison of LMWH batch to batch variation process and drug preparation according to a reference standard.

Fig. 1

Fig. 2a

Fig. 2b

Fig. 2c

# Fig 3a

## Fig 3b, section I

Intens. X $10^5$

| Peak Numer | Attribution |
|:---:|:---|
| 1 | Δ2,2,0 + 2,2,0 |
| 2 | Δ2,3,0 + 2,3,0 |
| 3 | Δ4,4,0 + 4,4,0 + A3,5,0 |
| 4 | Δ4,4,0-1,6an + Δ4,4,0 |
| 5 | Δ3,4,0 + U3,4,0 |
| 6 | Δ4,5,0 + 4,5,0 |
| 7 | Δ4,5,0 + Δ4,5,0-1,6an |
| 8 | Δ4,6,0 + 4,6,0 |
| 9 | Δ5,6,0 + 5,6,0 |
| 10 | Δ6,7,0 + 6,7,0 + Δ6,7,0-1,6an |
| 11 | ΔU7,3,3 + U7,3,3 |
| 12 | Δ6,8,0 + 6,8,0 + Δ5,7,0 |
| 13 | Δ6,8,0-1,6an |
| 14 | Δ6,9,0 + 6,9,0 |
| 15 | Δ8,11,0 + 8,11,0 |
| 16 | Δ9,7,3 + 9,7,3 |
| 17 | Δ8,12,0 + 8,12,0 |

## Fig 3b, section II

G5538 HA_RC5_01_4405,d: BPC 199-3001 –All MS, -Spectral Bigmd

| Peak Numer | Attribution |
|:---:|:---|
| 1 | A3,5,0 |
| 2 | Δ4,4,1 |
| 3 | ΔU3,4,0; Δ4,5,0; 4,5,0 |
| 4 | Δ4,5,0; 4,5,0 |
| 5 | Δ4,6,0; 4,6,0 |
| 6 | Δ4,6,0; 4,6,0 |

# Fig 3c

| Peak Numer | Attribution |
|:---:|:---|
| 1 | Δ5,4,1 |
| 2 | Δ6,5,1 |
| 3 | Δ6,5,1-1,6an |
| 4 | Δ5,5,1 |
| 5 | Δ6,6,1 +Δ4,6,0 |
| 6 | Δ6,6,1-1,6an (3 isomers) |
| 7 | A5,7,0-1.6an |
| 8 | Δ6,7,1 (3 isomers) |
| 9 | Δ6,7,1-1,6an (traces) |
| 10 | Δ8,9,1 (3 isomers) |
| 11 | Δ8,9,1-1,6an (2 isomers) |
| 12 | Δ8,10,1 (3 isomers) |
| 13 | Δ10,12,1 |
| 14 | Δ10,12,1-1,6an |

Fig. 4

Fig. 5

**Fig 6a**

**Fig 6b**

# Fig 7a, section I

| Fraction A$_0$ | |
|---|---|
| Peak number | Attribution |
| 1 | Δ6,4,1+1,6an/ Δ6,4,1/ A7,5,1 |
| 2 | Δ6,5,1/ A7,5,1/ Δ8,5,1 |
| 3-4 | Δ6,5,1/ A7,6,1/ Δ8,6,1 |
| 5 | A7,6,1+1,6an/ A7,6,1/ Δ8,8,1 |
| 6-7 | A7,6,1+1,6an/ Δ8,8,1 |
| 8 | Δ8,9,0 |
| 9 | Δ8,10,0+1,6an |
| 10 | Δ8,10,0 |
| 11 | Δ8,10,0+1,6an |
| 12 | Δ8,10,0 |
| 13 | Δ10,9,0+1,6an/ Δ12,7,5+1,6an |

| Fraction A$_1$ | |
|---|---|
| Peak number | Attribution |
| 1 | Δ6,7,1 |
| 2 | Δ6,7,1(or anche un SO3 in più) |
| 3 | Δ8,8,1 |
| 4 | Δ8,8,1-1,6an |
| 5 | Δ8,9,1 |
| 6 | Δ8,9,1-1,6an/ Δ7,7,1 |
| 7 | Δ8,10,1 |
| 8 | Δ8,10,0 |
| 9 | Δ8,10,0 |
| 10 | Δ8,11,0 |

## Fig 7a, section II

| Fraction A₀ | |
|---|---|
| Peak number | Attribution |
| 1 | Δ5,5,2 |
| 2 | A7,4,2/A7,4,4+1,6an |
| 3 | Δ8,4,2/Δ9,4,2 |
| 4 | Δ10,10,1 |
| 5-7 | Δ10,10,0/ Δ10,7,5+1,6an |

| Fraction A₁ | |
|---|---|
| Peak number | Attribution |
| 1 | Δ10,10,1 |
| 2 | Δ10,11,1 |
| 3 | Δ10,12,1 |

# Fig 7b, section I

| Fraction A[0] | |
|---|---|
| Peak Number | Attribution |
| 1 | Δ4,3,1-G-Gal-Gal-Xyl-Ser |
| 2 | Δ4,4,1-G-Gal-Gal-Xyl-Ser |
| 3 | Δ8,3,4 |
| 4-5 | Δ8,3,4/Δ8,6,1 |
| 6 | Δ8,6,1 |
| 7 | Δ8,9,1/ Δ8,7,0 |
| 8-9 | Δ8,9,1/ Δ8,10,0 |
| 10-11 | Δ8,11,0 |

| Fraction A[1] | |
|---|---|
| Peak Number | Attribution |
| 1 | Δ8,8,1 |
| 2 | A7,6,1 |
| 3-4 | Δ8,9,1 |
| 5-6 | Δ8,10,1 |
| 7-8 | Δ8,11,0 |

## Fig 7b, section II

| Fraction A0 | |
|---|---|
| Peak Number | Attribution |
| 1 | Δ10,2,5 |
| 2 | Δ10,3,4/ Δ10,3,5 |
| 3 | Δ10,4,4/ Δ10,3,4 |
| 4 | Δ10,5,4 |
| 5 | Δ10,6,4 |
| 6-7 | A9,7,1/ Δ10,10,1 |
| 8-9-10-11 | A9,7,1/ Δ10,10,1/ Δ10,10,5/ 12,4,3/ A11,14,0 |

| Fraction A1 | |
|---|---|
| Peak Number | Attribution |
| 1-2 | Δ10,10,1/ A9,7,1 |
| 3 | Δ10,11,1 |
| 4 | Δ10,12,1 |
| 5 | Δ10,12,1/ Δ10,12,5/ 12,4,3 |

## Fig 7c

| Fraction A$_0$ | |
|---|---|
| Peak number | Attribution |
| 1 | 8,8,1 aMan/8,6,0 aMan |
| 2 | 8,8,1 aMan/8,7,0 aMan/A9,8,0 aMan |
| 3 | 8,8,1 aMan/8,7,0 aMan |
| 4 | 8,7,0 aMan/ A9,8,0 aMan |
| 5 | 8,7,0 aMan/ A9,11,0 aMan/A7,11,0 aMan |
| 6-9 | 8,8,0 aMan/A9,11,0 aMan |
| 10 | 8,9,0 aMan/A9,12,0 aMan |
| 11 | A9,13,0 aMan/10,12,0 aMan/10,6,3 aMan |

| Fraction A$_1$ | |
|---|---|
| Peak number | Attribution |
| 1 | 8,8,1 aMan |
| 2 | A7,7,1 aMan/8,8,1 aMan |
| 3 | 8,8,1 aMan |
| 4 | A7,8,0 aMan |
| 5 | 8,9,0-aMan |
| 6 | 8,10,1-aMan |
| 7-8 | 8,10,0 aMan |
| 9-10 | A9,12,0-aMan 8,10,0-aMan |

Fig. 8

Fig. 9

Europäisches Patentamt

European Patent Office

Office européen des brevets

# EUROPEAN SEARCH REPORT

Application Number

EP 12 16 0613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BISIO A ET AL: "Structural features of low-molecular-weight heparins affecting their affinity to antithrombin", THROMBOSIS AND HAEMOSTASIS, SCHATTAUER GMBH, DE; US, vol. 102, no. 5, 1 November 2009 (2009-11-01), pages 865-873, XP002569660, ISSN: 0340-6245, DOI: 10.1160/TH09-02-0081 [retrieved on 2009-09-15] * abstract; figure 4 * * section 'materials and methods' * * page 866, left-hand column, last paragraph - right-hand column, paragraph 1 * * reference 22 (=D2). * | 1-10 | INV. G01N30/96 C08B37/00 C12Q1/56 G01N33/53 G01N33/66 |
| A | S. J. GOODGER ET AL: "Evidence That Heparin Saccharides Promote FGF2 Mitogenesis through Two Distinct Mechanisms", JOURNAL OF BIOLOGICAL CHEMISTRY, vol. 283, no. 19, 28 February 2008 (2008-02-28), pages 13001-13008, XP55034029, ISSN: 0021-9258, DOI: 10.1074/jbc.M704531200 * abstract * * Section 'preparation of heparin oligosaccharides on page 13002-13003 * | 1-5,7-10 | |

TECHNICAL FIELDS SEARCHED (IPC)

G01N
C08B
C12Q

-/--

The present search report has been drawn up for all claims

| Place of search | Date of completion of the search | Examiner |
|---|---|---|
| The Hague | 27 July 2012 | Mulder, Lonneke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 12 16 0613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | A. NAGGI: "Modulation of the Heparanase-inhibiting Activity of Heparin through Selective Desulfation, Graded N-Acetylation, and Glycol Splitting", JOURNAL OF BIOLOGICAL CHEMISTRY, vol. 280, no. 13, 17 November 2004 (2004-11-17), pages 12103-12113, XP55034072, ISSN: 0021-9258, DOI: 10.1074/jbc.M414217200 * abstract * * section experimental procedures * | 1-10 | |
| A,D | B. CASU; M. GUERRINI; A. NAGGI; M. PEREZ; G. TORRI; D. RIBATTI; P. CARMINATI; G. GIANNINI; S. PENCO; C. PISANO: "Short heparin sequences spaced by glycol-split uronate residues are antagonists of fibroblast growth factor 2 and angiogenesis inhibitors", BIOCHEMISTRY, vol. 41, 1 January 2002 (2002-01-01), pages 10519-10528, XP002492011, * abstract * * section Experimental Procedures * | 1-10 | |
| A,D | S. BERTINI; A. BISIO; G. TORRI; D. BENSI; M. TERBOJEVICH: "Molecular weight determination of heparin and dermatan sulfate by size exclusion chromatography with a triple detector array", BIOMACROMOL., vol. 6, 1 January 2005 (2005-01-01), pages 168-173, XP55033974, * abstract * * section "Experimental Section" * | 1-10 | TECHNICAL FIELDS SEARCHED (IPC) |

The present search report has been drawn up for all claims

| Place of search | Date of completion of the search | Examiner |
|---|---|---|
| The Hague | 27 July 2012 | Mulder, Lonneke |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7790466 B1

### Non-patent literature cited in the description

- **B. CASU ; U. LINDAHL.** Structure and biological interactions of heparin and heparan sulfate. *Advances Carbohydr. Chem. Biochem.,* 2001, vol. 57, 159-206
- **R.J. LINHARDT.** Heparin: structure and activity. [2003 Claude S. Hudson Award address in carbohydrate chemistry. *J. Med. Chem.,* 2003, 2551-2564
- Structure and active domains of heparin. **B. CASU.** Chemistry and Biology of Heparin and Heparan Sulfate. Elsevier, 2005, 1-28
- **H.E. CONRAD.** Heparin binding proteins. Academic Press, 2003
- **I CAPILA ; R.J. LINHARDT.** Heparin-protein interactions. *Angew. Chem. Int. Ed.,* 2002, vol. 41, 390-412
- **J.M. WHITELOCK ; R.V. LOZZO.** Heparan sulfate: a complex polymer charged with biological activity. *Chem. Rev.,* 2005, vol. 105, 2745-2764
- **J.R. BISHOP ; M. SCHUKSK ; J.D. ESKO.** Heparan sulfate proteoglycans fine-tune mammalian physiology. *Nature,* 2007, vol. 446, 1030-1037
- **M. GUERRINI ; S. GUGLIERI ; B. CASU ; G. TORRI ; P. MOURIER ; C. BOUDIER ; C. VISKOV.** Antithrombin-binding octasaccharides and role of extensions of the active pentasaccharide sequence in the specificity and strength of interaction. Evidence for very high affinity induced by an unusual glucuronic acid residue. *J Biol Chem.,* 2008, vol. 26, 26662-26675
- **M. GUERRINI ; S. ELLI ; D. GAUDESI ; G. TORRI ; B. CASU ; P. MOURIER ; F. HERMAN ; C. BOUDIER ; M. LORENZ ; C. VISKOV.** Effects on molecular conformation and anticoagulant activities of 1,6-anhydrosugars at the reducing terminal of antithrombin-binding octasaccharides isolated from low-molecular-weight heparin enoxaparin. *Med. Chem.*
- **M. GUERRINI ; S. GUGLIERI ; D. BECCATI ; G. TORRI ; C. VISKOV ; P. MOURIER.** Conformational transitions induced in heparin oligosaccharides by binding with antithrombin III. *Biochem. J.,* 2006, vol. 15, 191-198
- **A. BISIO ; A. MANTEGAZZA ; E. URSO ; A. NAGGI ; G. TORRI ; C. VISKOV ; B. CASU.** High-performance liquid chromatographic/mass spectrometric studies on the susceptibility of heparin species to cleavage by heparanase. *Semin. Thromb. Hemost.,* 2007, vol. 33, 488-495
- **M. PETITOU ; C. A. VAN BOECKEL.** A Synthetic Antithrombin III Binding Pentasaccharide Is Now a Drug! What Comes Next?. *Angew. Chem. Int. Ed.,* 2004, vol. 43 (24), 3118-3133
- **HÖÖK M ; BJÖRK I ; HOPWOOD J et al.** Anticoagulant activity of heparin: separation of high-activity and low-activity heparin species by affinity chromatography on immobilized antithrombin. *FEBS Lett.,* 1976, vol. 66, 90-93
- Methods for structural analysis of heparin and heparan sulfate. **I. CAPILA ; N.S. GUNAY ; Z. SHRIVER ; G. VENKATARAMAN.** Chemistry and biology of heparin and heparan sulfate. Elsevier, 2005, 55-78
- **B. CASU ; P. ORESTE ; G. TORRI ; G. ZOPPETTI ; J.CHOAY ; J.C. LORMEAU ; M. PETITOU ; P. SINAÿ.** The structure of heparin oligosaccharide fragments with high anti-(factor Xa) activity containing the minimal antithrombin III-binding sequence. Chemical and 13C-NMR studies. *Biochem. J.,* 1981, vol. 197, 599-609
- **M. SUNDARAM ; Y. QI ; Z. SHRIVER ; D. LIU ; G. ZHAO ; G. VENKATARAMAN ; R. LANGER ; R. SASISEKHARAN.** Rational design of low-molecular weight heparins with improved in vivo activity. *Proc. Natl Acad. Sci. USA,* 2003, vol. 100, 651-656
- **R.M. MARKS ; H. LU ; R. SUNDARESAN ; T. TOIDA ; A. SUZUKI ; T. IMANARI ; M.J. HERNÁIZ ; R.J. LINHARDT.** Probing the interaction of dengue virus envelope protein with heparin: assessment of glycosaminoglycan-derived Inhibitors. *J. Med. Chem.,* 2001, vol. 44, 2178-2187
- **M. GUERRINI ; S. GUGLIERI ; A. NAGGI ; R. SASISEKHARAN ; G. TORRI.** Low molecular weight heparins: structural differentiation by bidimensional nuclear magnetic resonance spectroscopy. *Semin. Thromb. Hemost.,* 2007, vol. 33, 478-487

- **P.A.J. MOURIER ; C. VISKOV.** Chromatographic analysis and sequencing approach of heparin oligosaccharides using cetyltrimethylammonium dynamically coated stationary phases. *Anal. Biochem.,* 2004, vol. 332, 299-313
- **M. KUSCHE ; G. TORRI ; B. CASU ; U. LINDAHL.** Biosynthesis of heparin. Availability of glucosaminyl-3-O-sulfation sites. *J. Biol. Chem.,* 1990, vol. 265, 7292-7300
- **A. BISIO ; D. VECCHIETTI ; L. CITTERIO ; M. GUERRINI ; R. RAMAN ; S. BERTINI ; G. EISELE ; A. NAGGI ; R. SASISEKHARAN ; G. TORRI.** Structural features of low molecular weight heparins affecting their affinity to antithrombin. *Thromb. Haemost.,* 2009, vol. 102, 865-873
- **E. VISMARA ; M.PIERINI ; S. GUGLIERI ; L. LIVERANI ; G.MASCELLANI ; G. TORRI.** Structural modification induced in heparin by a Fenton-type depolymerization process. *Semin. Thromb. Hemost.,* 2007, vol. 33, 466-477
- **B. CASU ; M. GUERRINI ; A. NAGGI ; M. PEREZ ; G. TORRI ; D. RIBATTI ; P. CARMINATI ; G. GIANNINI ; S. PENCO ; C. PISANO.** Short heparin sequences spaced by glycol-split uronate residues are antagonists of fibroblast growth factor 2 and angiogenesis inhibitors. *Biochemistry,* 2002, vol. 41, 10519-10528
- **B. CASU ; G. TORRI.** Structural characterization of low molecular weight heparins. *Semin. Thromb. Hemost.,* 1999, vol. 25, 17-25
- **M. GUERRINI ; A. BISIO ; G. TORRI.** Combined quantitative H and C nuclear magnetic resonance spectroscopy for characterization of heparin preparations. *Semin. Thromb. Hemost.,* 2001, vol. 27, 473-482
- **A. BISIO ; S. GUGLIERI ; M. FRIGERIO ; G. TORRI ; E. VISMARA ; U. CORNELLI ; D. BENSI ; S. GONELLA ; L. DE AMBROSI.** Controlled γ-ray irradiation of heparin generates oligosaccharides enriched in highly sulfated sequences. *Carbohydr. Polym.,* 2004, vol. 55, 101-112
- **M. GUERRINI ; A. NAGGI ; S. GUGLIERI ; R. SANTARSIERO ; G. TORRI.** Complex glycosaminoglycans: profiling substitution patterns by two-dimensional nuclear magnetic resonance spectroscopy. *Anal. Biochem.,* 2005, vol. 337, 35-47
- **G. MASCELLANI ; M. GUERRINI ; G. TORRI ; L. LIVERANI ; F. SPELTA ; P. BIANCHINI.** Characterization of di- and monosulfated, unsaturated heparin disaccharides with terminal N-sulfated 1,6-anhydro-β-D-glucosamine or N-sulfated 1,6-anhydro-β-D-mannosamine residues. *Carbohydr. Res.,* 2007, vol. 342, 835-842
- **M. HRICOVINI ; M. GUERRINI ; G. TORRI ; S. PIANI ; F. UNGARELLI.** Conformational analysis of heparin epoxide in aqueous solution. An NMR relaxation study. *Carbohydr. Res.,* 1995, vol. 277, 11-23
- **S. BERTINI ; A. BISIO ; G. TORRI ; D. BENSI ; M. TERBOJEVICH.** Molecular weight determination of heparin and dermatan sulfate by size exclusion chromatography with a triple detector array. *Biomacromol.,* 2005, vol. 6, 168-173
- **T. BITTER ; H.M. MUIR.** Quantitative determination of uronic acids with m-hydroxydiphenyl. *Anal. Biochem.,* 1962, vol. 4, 330-334
- Separation and sequencing of heparin and heparin sulphate saccharides. **M.A. SKIDMORE ; J.E. TURNBULL.** Chemistry and Biology of Heparin and Heparan Sulfate. Elsevier Ltd, 2006, 179-201
- **A. NAGGI ; B. CASU ; M. PEREZ ; G. TORRI ; G. CASSINELLI ; S. PENCO ; C. PISANO ; G. GIANNINI ; R. ISHAI-MICHAELI ; I. VLODAVSKY.** Modulation of the heparanase-inhibiting activity of heparin through selective desulfation, graded N-acetylation, and glycol-splitting. *J. Biol. Chem.,* 2005, vol. 280, 12130-12113